(19) Europäisches Patentamt
European Patent Office
Office européen des brevets

(11) EP 3 598 135 A1

(12) **EUROPEAN PATENT APPLICATION**

(43) Date of publication:
**22.01.2020 Bulletin 2020/04**

(51) Int Cl.:
***G01N 33/68*** *(2006.01)*

(21) Application number: **18184710.4**

(22) Date of filing: **20.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Univerzita Palackého v Olomouci**
**771 47 Olomouc (CZ)**

(72) Inventors:
• **HRUSKA, Miroslav**
**02201 Cadca (SK)**
• **HAJDUCH, Marian**
**79305 Moravsky Beroun (CZ)**
• **DZUBAK, Petr**
**75103 Brodek u Prerova (CZ)**

(74) Representative: **Hartvichova, Katerina et al**
**HARBER IP s.r.o.**
**Dukelskych hrdinu 567/52**
**170 00 Praha 7 (CZ)**

Remarks:
The references to the drawing no. Figure 42 is deemed to be deleted (Rule 56(4) EPC).

(54) **METHOD OF IDENTIFICATION OF ENTITIES FROM MASS SPECTRA**

(57) The present invention relates to a method for determination of identity of at least one entity from a mass spectrum of said at least one entity and optionally from additional data from chemical, physical, biochemical or biological analysis of said at least one entity, for each entity comprising the steps of:

a) collecting analytical data from mass spectrum of the entity, and optionally collecting additional analytical data from a chemical, physical, biochemical or biological analysis of the entity,

b) obtaining a plurality of candidate identities of the entity and obtaining the prevalences of said candidate identities of the entity, whereas for each candidate identity it applies that all candidate identities with a higher prevalence are included in the plurality of candidate identities;

c) for each candidate identity of an entity, calculation of its score, said calculation involving at least prevalence of entity, or at least prevalence of entity and agreement with mass spectrum,

d) determining the identity of an entity as the candidate identity with the score closest to the score which would correspond to the true identity of the entity.

The entity may be any chemical or biological entity, in particular a peptide, a protein, a lipid, a nucleic acid, a metabolite or a small molecule.

The determination of the identity of the entity solves the problems of interpretation of mass spectra commonly encountered in shotgun proteomics and many other fields.

The method of the present invention may be also used for determination of identity, in particular for authentication of cell lines or identification of an individual from mass spectra of proteome.

The method may be also used for identification of non-host organism from mass spectra of proteome of host organism, in particular for diagnosis of microbial infection or colonization.

The method may be also used for identification of presence of a tumour from mass spectra of body fluid proteins or estimation of tumour characteristics through presence or absence of somatic mutations.

The method may be also used for monitoring of organ transplantation and early detection of transplant rejection from mass spectra of biological materials of recipient.

SEQUENCE LISTING

<110> Univerzita Palackeho v Olomouci

<120> Method of identification of entities from mass spectra

<130> P

<160> 131

<170> PatentIn version 3.5

<210> 1
<211> 11
<212> PRT
<213> Artificial

<220>
<223> candidate identity

<400> 1

Arg Phe Tyr Pro His Asp Val Leu Ser Leu Pro
1               5                   10


<210> 2
<211> 10
<212> PRT
<213> Artificial

<220>
<223> candidate identity

<400> 2

Ser Met Val Glu Leu Trp Ala Trp Arg Glu
1               5                   10


<210> 3
<211> 13
<212> PRT
<213> artificial

<220>
<223> candidate identity

<400> 3

Arg Ser Pro Ser Gly Phe Gly Asp Pro Gly Lys Lys Asp
1               5                   10


<210> 4
<211> 13
<212> PRT
<213> Artificial

<220>
<223> candidate identity

<400> 4

Arg Met Thr Gln Ala Leu Ala Leu Gln Ala Gly Ser Leu
1               5                   10

<210>   5
<211>   11
<212>   PRT
<213>   artificial

<220>
<223>   candidate identity

<400>   5

Asp Asn Thr Val Val Met Glu Glu Ile Arg Arg
1               5                   10

<210>   6
<211>   12
<212>   PRT
<213>   artificial

<220>
<223>   candidate identity

<400>   6

Thr Ala Ile Ser Asn Ala Ser Asp Val Trp Lys Lys
1               5                   10

<210>   7
<211>   11
<212>   PRT
<213>   artificial

<220>
<223>   candidate identity

<400>   7

Arg Ala Asn Ala Phe Leu Glu Glu Leu Arg Pro
1               5                   10

<210>   8
<211>   12
<212>   PRT
<213>   artificial

<220>
<223>   candidate identity

<400>   8

Lys Met Thr Thr Gln Ala Leu Glu Glu Leu Arg Ser
1               5                   10

<210>   9
<211>   14

```
<212>  PRT
<213>  artificial

<220>
<223>  candidate identity

<400>  9

Arg Thr Ser Gly Gly Ala Gly Gly Leu Gly Ser Leu Arg Ala
1               5                   10


<210>  10
<211>  11
<212>  PRT
<213>  artificial

<220>
<223>  candidate identity

<400>  10

Lys Met Ala Gln Ala Leu Glu Glu Leu Arg Ser
1               5                   10


<210>  11
<211>  13
<212>  PRT
<213>  artificial

<220>
<223>  candidate identity

<400>  11

Arg Ser Leu Met Met Val Asp Ile Ala Glu Gly Arg Lys
1               5                   10


<210>  12
<211>  13
<212>  PRT
<213>  artificial

<220>
<223>  candidate identity

<400>  12

Lys Leu Val Met Met Asp Ser Ile Ala Glu Gly Arg Ile
1               5                   10


<210>  13
<211>  12
<212>  PRT
<213>  artificial

<220>
<223>  candidate identity

<400>  13
```

Arg Thr Met Gln Ala Leu Glu Ile Glu Arg Arg Gln
1               5               10

<210>  14
<211>  11
<212>  PRT
<213>  artificial

<220>
<223>  candidate identity

<400>  14

Met Ala Ser Ser Leu Leu Ala Gly Glu Arg Leu
1               5               10

<210>  15
<211>  11
<212>  PRT
<213>  artificial

<220>
<223>  candidate identity

<400>  15

Lys Asp Cys Val Leu Asn Ala Thr Leu Lys Gln
1               5               10

<210>  16
<211>  11
<212>  PRT
<213>  artificial

<220>
<223>  candidate identity

<400>  16

Lys Asp Cys Val Leu Asn Ala Thr Leu Lys Gln
1               5               10

<210>  17
<211>  11
<212>  PRT
<213>  artificial

<220>
<223>  candidate identity

<400>  17

Lys Met Ala Gln Ala Leu Glu Glu Leu Arg Ser
1               5               10

<210>  18
<211>  15

```
<212>   PRT
<213>   artificial

<220>
<223>   candidate identity

<400>   18

Arg Thr Ser Asp Asp Gly Ala Gly Gly Leu Gly Ser Leu Arg Ala
1               5                   10                  15


<210>   19
<211>   6
<212>   PRT
<213>   artificial

<220>
<223>   variant peptide

<400>   19

Asn Glu Ile Pro Ile Arg
1               5


<210>   20
<211>   13
<212>   PRT
<213>   artificial

<220>
<223>   variant peptide

<400>   20

Ala Ala Val Ala Ala Ile Thr Gln Ala Leu Val Gly Arg
1               5                   10


<210>   21
<211>   13
<212>   PRT
<213>   artificial

<220>
<223>   variant peptide

<400>   21

Ser Pro Pro Leu Pro Gly Asp Leu Gly Gly Pro Ser Lys
1               5                   10


<210>   22
<211>   44
<212>   PRT
<213>   artificial

<220>
<223>   variant peptide

<400>   22
```

Leu Ser Ala Ala Gln Thr Asn Gly Gly Gly Ser Ala Gly Met Glu Gly
1                   5                   10                  15

Ile Met Asn Pro Tyr Thr Ala Leu Pro Thr Pro Gln Gln Leu Leu Ala
                20                  25                  30

Ile Glu Gln Ser Val Tyr Ser Ser Asp Pro Phe Arg
            35                  40

<210> 23
<211> 23
<212> PRT
<213> artificial

<220>
<223> variant peptide

<400> 23

Asn Thr Glu Ile Leu Thr Gly Ser Trp Ser Asp Gln Thr Tyr Pro Glu
1                   5                   10                  15

Gly Thr His Ala Ile Tyr Lys
                20

<210> 24
<211> 12
<212> PRT
<213> artificial

<220>
<223> candidate identity

<400> 24

Gln Pro Thr His Gln Gly Gly Pro Ala Gln Asp Arg
1                   5                   10

<210> 25
<211> 12
<212> PRT
<213> artificial

<220>
<223> candidate identity

<400> 25

Gly Gly Gly Gly Ser Ala Arg Tyr Asn Gln Gly Arg
1                   5                   10

<210> 26
<211> 12
<212> PRT
<213> artificial

```
<220>
<223>  candidate identity

<400>  26

Gly Gly Gly Gly Ser Ala Arg Tyr Asn Gln Gly Arg
1               5                   10


<210>  27
<211>  12
<212>  PRT
<213>  artificial

<220>
<223>  candidate identity

<400>  27

Gly Asn Pro Ser Gly Gly Gly Asn Val Gln His Arg
1               5                   10


<210>  28
<211>  10
<212>  PRT
<213>  artificial

<220>
<223>  candidate identity

<400>  28

Gln Gln Glu Leu Thr His Gln Glu His Arg
1               5                   10


<210>  29
<211>  29
<212>  PRT
<213>  artificial

<220>
<223>  candidate identity

<400>  29

Gly Ser Gly Asp Pro Ser Ser Ser Ser Ser Ser Gly Asn Pro Ile Val
1               5                   10                      15


Tyr Leu Asp Val Asp Ala Asn Gly Lys Pro Leu Gly Arg
            20                  25


<210>  30
<211>  18
<212>  PRT
<213>  artificial

<220>
<223>  candidate identity
```

<400> 30

Ile Gly Leu Ile Gly Gly Thr Gly Leu Asp Asp Pro Glu Ile Leu Glu
1               5                   10                  15

Gly Arg


<210> 31
<211> 21
<212> PRT
<213> artificial

<220>
<223> candidate identity

<400> 31

Val Glu Gly Thr Glu Pro Thr Thr Ala Phe Asn Ile Phe Val Gly Asn
1               5                   10                  15

Leu Asn Phe Asn Lys
                20


<210> 32
<211> 19
<212> PRT
<213> artificial

<220>
<223> candidate identity

<400> 32

Gly Glu Glu Leu Gly Gly Gly Gln Asp Pro Val Gln Leu Ile Ser Gly
1               5                   10                  15

Phe Pro Arg


<210> 33
<211> 17
<212> PRT
<213> artificial

<220>
<223> candidate identity

<400> 33

His Pro Leu His Val Thr Tyr Ala Gly Ala Ala Val Asp Glu Leu Gly
1               5                   10                  15

Lys

```
<210>   34
<211>   20
<212>   PRT
<213>   artificial

<220>
<223>   candidate identity

<400>   34

Val Pro Ile Ala Ser Gln Gly Leu Gly Pro Gly Ser Thr Val Leu Leu
1               5                   10                  15


Val Val Asp Lys
            20


<210>   35
<211>   21
<212>   PRT
<213>   artificial

<220>
<223>   candidate identity

<400>   35

Gln Ser Gly Gly Ser Ser Gln Ala Gly Ala Val Thr Val Ser Asp Val
1               5                   10                  15


Gln Glu Leu Met Arg
            20


<210>   36
<211>   18
<212>   PRT
<213>   artificial

<220>
<223>   candidate identity

<400>   36

Asp Pro Gly Gly Leu Thr Ala Gly Ser Thr Asp Glu Pro Pro Met Leu
1               5                   10                  15


Thr Lys


<210>   37
<211>   18
<212>   PRT
<213>   artificial

<220>
<223>   candidate identity
```

<400> 37

Asp Pro Gly Gly Leu Thr Ala Gly Ser Thr Asp Glu Pro Pro Met Leu
1               5                   10                  15

Thr Lys


<210> 38
<211> 24
<212> PRT
<213> artificial

<220>
<223> candidate identity

<400> 38

Asn Phe Tyr Gly Gly Asn Gly Ile Val Gly Ala Gln Val Pro Leu Gly
1               5                   10                  15

Ala Gly Ile Ala Leu Ala Cys Lys
                20


<210> 39
<211> 17
<212> PRT
<213> artificial

<220>
<223> candidate identity

<400> 39

His Pro Leu His Val Thr Tyr Ala Gly Ala Ala Val Asp Glu Leu Gly
1               5                   10                  15

Lys


<210> 40
<211> 20
<212> PRT
<213> artificial

<220>
<223> candidate identity

<400> 40

Thr Val Ser Gly Thr Cys Gly Pro Gly Gln Pro Ala Ser Ser Ser Gly
1               5                   10                  15

Gly Pro Gly Arg
                20

```
<210>  41
<211>  21
<212>  PRT
<213>  artificial

<220>
<223>  candidate identity

<400>  41

Asn Ser Asn Pro Val Ile Ala Glu Leu Ser Gln Ala Ile Asn Ser Gly
1               5                   10                  15


Thr Leu Leu Ser Lys
                20


<210>  42
<211>  17
<212>  PRT
<213>  artificial

<220>
<223>  candidate identity

<400>  42

Val Trp Leu Asp Pro Asn Glu Thr Asn Glu Leu Ala Asn Ala Asn Ser
1               5                   10                  15


Arg



<210>  43
<211>  29
<212>  PRT
<213>  artificial

<220>
<223>  candidate identity

<400>  43

Gly Ser Gly Asp Pro Ser Ser Ser Ser Ser Ser Gly Asn Pro Ile Val
1               5                   10                  15


Tyr Leu Asp Val Asp Ala Asn Gly Lys Pro Leu Gly Arg
                20                  25


<210>  44
<211>  21
<212>  PRT
<213>  artificial

<220>
<223>  candidate identity
```

<400> 44

Val Glu Gly Thr Glu Pro Thr Thr Ala Phe Asn Ile Phe Val Gly Asn
1               5                   10                  15

Leu Asn Phe Asn Lys
                20


<210> 45
<211> 19
<212> PRT
<213> artificial

<220>
<223> candidate identity

<400> 45

Gly Glu Glu Leu Gly Gly Gly Gln Asp Pro Val Gln Leu Ile Ser Gly
1               5                   10                  15

Phe Pro Arg


<210> 46
<211> 20
<212> PRT
<213> artificial

<220>
<223> candidate identity

<400> 46

Val Pro Ile Ala Ser Gln Gly Leu Gly Pro Gly Ser Thr Val Leu Leu
1               5                   10                  15

Val Val Asp Lys
                20


<210> 47
<211> 18
<212> PRT
<213> artificial

<220>
<223> candidate identity

<400> 47

Asp Pro Gly Gly Leu Thr Ala Gly Ser Thr Asp Glu Pro Pro Met Leu
1               5                   10                  15

Thr Lys

47

```
<210>   48
<211>   15
<212>   PRT
<213>   artificial

<220>
<223>   candidate identity

<400>   48

Val Glu His Leu Glu Glu Gly Pro Met Leu Glu Gln Leu Ser Lys
1               5                   10                  15


<210>   49
<211>   14
<212>   PRT
<213>   artificial

<220>
<223>   candidate identity

<400>   49

Ile Val Val Glu Cys Val Met Asn Asn Val Thr Cys Thr Arg
1               5                   10


<210>   50
<211>   19
<212>   PRT
<213>   artificial

<220>
<223>   candidate identity

<400>   50

Gly Val Glu Ala Ala Asn Val Thr Gly Pro Gly Gly Val Pro Val Gln
1               5                   10                  15


Gly Ser Lys


<210>   51
<211>   14
<212>   PRT
<213>   artificial

<220>
<223>   candidate identity

<400>   51

Asp Met Leu Leu Glu Ile Glu Glu Gln Leu Ala Glu Ser Arg
1               5                   10


<210>   52
```

```
<211>   14
<212>   PRT
<213>   artificial

<220>
<223>   candidate identity

<400>   52

Ile Val Val Glu Cys Val Met Asn Asn Val Thr Cys Thr Arg
1               5                   10


<210>   53
<211>   20
<212>   PRT
<213>   artificial

<220>
<223>   candidate identity

<400>   53

Val Asn Asn Ser Ser Leu Ile Gly Leu Gly Tyr Thr Gln Thr Ile Lys
1               5                   10                  15

Pro Gly Ile Lys
            20


<210>   54
<211>   14
<212>   PRT
<213>   artificial

<220>
<223>   candidate identity

<400>   54

Ser Pro Ala Ser Asp Thr Tyr Ile Val Phe Gly Glu Val Lys
1               5                   10


<210>   55
<211>   14
<212>   PRT
<213>   artificial

<220>
<223>   candidate identity

<400>   55

Ile Val Val Glu Cys Val Met Asn Asn Val Thr Cys Thr Arg
1               5                   10


<210>   56
<211>   14
<212>   PRT
<213>   artificial
```

<220>
<223> candidate identity

<400> 56

Ile Val Val Glu Cys Val Met Asn Asn Val Thr Cys Thr Arg
1               5                   10


<210> 57
<211> 17
<212> PRT
<213> artificial

<220>
<223> candidate identity

<400> 57

Ile Glu Ala Gly Glu Gly Glu Thr Ala Val Leu Asn Gln Leu Gln Glu
1               5                   10                  15


Lys


<210> 58
<211> 24
<212> PRT
<213> artificial

<220>
<223> candidate identity

<400> 58

Asp Met Asp Leu Thr Gly Cys Leu Glu Ser Gly Gly Ser Glu Glu Pro
1               5                   10                  15


Gly Gly Ile Gly Val Gly Glu Lys
                20


<210> 59
<211> 23
<212> PRT
<213> artificial

<220>
<223> candidate identity

<400> 59

Leu Ser Tyr Asp Gln Gln Gln Gln Gln Gln Gln Gln Gln Gln Gln
1               5                   10                  15


Gln Gln Ala Ile Gln Ser Arg
                20

```
<210>    60
<211>    19
<212>    PRT
<213>    artificial

<220>
<223>    candidate identity

<400>    60

Gly Glu Glu Leu Gly Gly Gly Gln Asp Pro Val Gln Leu Ile Ser Gly
1               5                   10                  15


Phe Pro Arg



<210>    61
<211>    20
<212>    PRT
<213>    artificial

<220>
<223>    candidate identity

<400>    61

Val Pro Ile Ala Ser Gln Gly Leu Gly Pro Gly Ser Thr Val Leu Leu
1               5                   10                  15


Val Val Asp Lys
            20



<210>    62
<211>    18
<212>    PRT
<213>    artificial

<220>
<223>    candidate identity

<400>    62

Asp Pro Gly Gly Leu Thr Ala Gly Ser Thr Asp Glu Pro Pro Met Leu
1               5                   10                  15


Thr Lys



<210>    63
<211>    17
<212>    PRT
<213>    artificial

<220>
<223>    candidate identity
```

<400> 63

Val Trp Leu Asp Pro Asn Glu Thr Asn Glu Leu Ala Asn Ala Asn Ser
1               5                   10                  15

Arg


<210> 64
<211> 17
<212> PRT
<213> artificial

<220>
<223> candidate identity

<400> 64

Ala Leu Asp Val Gly Ser Gly Ser Gly Leu Leu Thr Ala Cys Phe Ala
1               5                   10                  15

Arg


<210> 65
<211> 18
<212> PRT
<213> artificial

<220>
<223> candidate identity

<400> 65

Asn Ser Gly Pro Ile Gly Thr Glu Met Asn Thr Gly Phe Ser Ser Glu
1               5                   10                  15

Val Lys


<210> 66
<211> 15
<212> PRT
<213> artificial

<220>
<223> candidate identity

<400> 66

Phe Leu Gln Glu His Gly Ser Asp Ser Phe Ile Ala Glu His Lys
1               5                   10                  15


<210> 67
<211> 14
<212> PRT

52

<213> artificial

<220>
<223> candidate identity

<400> 67

Thr Ala Met Asn Val Asn Glu Ile Phe Met Ala Leu Ala Lys
1               5                   10


<210> 68
<211> 15
<212> PRT
<213> artificial

<220>
<223> candidate identity

<400> 68

Ser Cys Gln Thr Ala Leu Val Glu Ile Leu Asp Val Ile Ala Arg
1               5                   10                  15


<210> 69
<211> 15
<212> PRT
<213> artificial

<220>
<223> candidate identity

<400> 69

Trp Ser Gly Pro Leu Ser Leu Gln Glu Val Asp Glu Gln Pro Lys
1               5                   10                  15


<210> 70
<211> 15
<212> PRT
<213> artificial

<220>
<223> candidate identity

<400> 70

Asn Leu Thr Asn Pro Asn Thr Val Ile Ile Ile Ile Gly Asn Lys
1               5                   10                  15


<210> 71
<211> 13
<212> PRT
<213> artificial

<220>
<223> candidate identity

<400> 71

Tyr Gly Tyr Thr His Ile Ser Ala Gly Glu Leu Leu Arg
1               5                   10

<210>   72
<211>   17
<212>   PRT
<213>   artificial

<220>
<223>   candidate identity

<400>   72

Thr Val Asn Glu Leu Gln Asn Leu Thr Ala Thr Glu Val Val Val Pro
1               5                   10                  15

Arg

<210>   73
<211>   16
<212>   PRT
<213>   artificial

<220>
<223>   candidate identity

<400>   73

Thr Ile Leu Asp Gln His Gly Gln Tyr Pro Ile Trp Met Asn Gln Arg
1               5                   10                  15

<210>   74
<211>   15
<212>   PRT
<213>   artificial

<220>
<223>   candidate identity

<400>   74

Ala Val Phe Val Asp Leu Glu Pro Thr Val Ile Asp Asp Ile Arg
1               5                   10                  15

<210>   75
<211>   20
<212>   PRT
<213>   artificial

<220>
<223>   candidate identity

<400>   75

Ala Ala Glu Leu Leu Ala Asn Ser Leu Ala Thr Ala Gly Asp Gly Leu
1               5                   10                  15

Ile Glu Leu Arg
20


<210> 76
<211> 10
<212> PRT
<213> artificial

<220>
<223> candidate identity

<400> 76

Gly Pro Glu Val Asp Val Asn Leu Pro Lys
1               5               10


<210> 77
<211> 9
<212> PRT
<213> artificial

<220>
<223> candidate identity

<400> 77

Glu Ile Glu Asn Ile Gln Thr Tyr Arg
1               5


<210> 78
<211> 16
<212> PRT
<213> artificial

<220>
<223> candidate identity

<400> 78

Gly Ala Ala Gly Ser Gly Asp Ser Ala Ala Ala Ala Glu Trp Ile Arg
1               5               10              15


<210> 79
<211> 12
<212> PRT
<213> artificial

<220>
<223> candidate identity

<400> 79

Thr Ile Leu Thr Leu Thr Gly Val Ser Thr Leu Arg
1               5               10


<210> 80
<211> 11

<212> PRT
<213> artificial

<220>
<223> candidate identity

<400> 80

Ile Ile Ile Glu Asp Leu Leu Glu Ala Thr Arg
1               5               10

<210> 81
<211> 16
<212> PRT
<213> artificial

<220>
<223> candidate identity

<400> 81

Gly Ser Leu Gly Asp Arg Pro Glu Ala Ser Met Thr Pro Asp Ala Lys
1               5               10                  15

<210> 82
<211> 33
<212> PRT
<213> artificial

<220>
<223> candidate identity

<400> 82

Gly Leu Val Glu Pro Val Asn Met Val Asp Asn Gly Asp Gly Thr His
1               5               10                  15

Thr Val Thr Tyr Thr Pro Ser Gln Glu Gly Pro Tyr Met Val Ser Val
                20                  25                  30

Lys

<210> 83
<211> 30
<212> PRT
<213> artificial

<220>
<223> candidate identity

<400> 83

Val Gly Glu Ala Gly Leu Leu Ser Val Asn Cys Ser Glu Ala Gly Pro
1               5               10                  15

Gly Ala Leu Gly Leu Glu Ala Val Ser Asp Ser Gly Thr Lys

20                          25                          30

```
<210>   84
<211>   15
<212>   PRT
<213>   artificial

<220>
<223>   candidate identity

<400>   84

Gln Val Val Ser Ala Val Thr Ala Leu Val Glu Ala Ala Glu Arg
1               5                   10                  15


<210>   85
<211>   9
<212>   PRT
<213>   artificial

<220>
<223>   candidate identity

<400>   85

Tyr Ile Glu Thr Asp Pro Ala Asn Arg
1               5


<210>   86
<211>   9
<212>   PRT
<213>   artificial

<220>
<223>   candidate identity

<400>   86

Val Gly Asp Thr Tyr Glu Arg Pro Lys
1               5


<210>   87
<211>   11
<212>   PRT
<213>   artificial

<220>
<223>   candidate identity

<400>   87

Asp Ile Phe Thr Gly Leu Ile Gly Pro Met Lys
1               5                   10


<210>   88
<211>   16
<212>   PRT
<213>   artificial
```

```
<220>
<223>   candidate identity

<400>   88

Ser Gly Ile Pro Ile Val Thr Ser Pro Tyr Gln Ile His Phe Thr Lys
1               5                   10                  15


<210>   89
<211>   15
<212>   PRT
<213>   artificial

<220>
<223>   candidate identity

<400>   89

Lys Leu Trp Ala Tyr Leu Thr Ile Asn Gln Leu Leu Ala Glu Arg
1               5                   10                  15


<210>   90
<211>   16
<212>   PRT
<213>   artificial

<220>
<223>   candidate identity

<400>   90

Arg Phe Asp Glu Ile Leu Glu Ala Ser Asp Gly Ile Met Val Ala Arg
1               5                   10                  15


<210>   91
<211>   20
<212>   PRT
<213>   artificial

<220>
<223>   candidate identity

<400>   91

Leu Leu Cys Asn Gly Asp Asn Asp Cys Gly Asp Gln Ser Asp Glu Ala
1               5                   10                  15


Asn Cys Arg Arg
                20


<210>   92
<211>   10
<212>   PRT
<213>   artificial

<220>
<223>   candidate identity
```

```
<400>    92

Ile His Gly Ile Leu Ser Asn Thr His Arg
1               5                   10


<210>    93
<211>    10
<212>    PRT
<213>    artificial

<220>
<223>    candidate identity

<400>    93

Glu Val Tyr Gly Phe Asn Pro Glu Gly Lys
1               5                   10


<210>    94
<211>    9
<212>    PRT
<213>    artificial

<220>
<223>    candidate identity

<400>    94

Val Asn Leu Gly Val Gly Ala Tyr Arg
1               5


<210>    95
<211>    11
<212>    PRT
<213>    artificial

<220>
<223>    candidate identity

<400>    95

Val Gln Phe Glu Leu His Tyr Gln Glu Val Lys
1               5                   10


<210>    96
<211>    9
<212>    PRT
<213>    artificial

<220>
<223>    candidate identity

<400>    96

Thr Asn Ser Leu Tyr Val Ser Glu Arg
1               5
```

```
<210>  97
<211>  10
<212>  PRT
<213>  artificial

<220>
<223>  candidate identity

<400>  97

Thr Ser Gln Val Ile Glu Tyr Gln Leu Lys
1               5                   10


<210>  98
<211>  13
<212>  PRT
<213>  artificial

<220>
<223>  candidate identity

<400>  98

Gln Glu Asp Val Ser Val Ser Gln Gly Gln Trp Asp Lys
1               5                   10


<210>  99
<211>  13
<212>  PRT
<213>  artificial

<220>
<223>  candidate identity

<400>  99

Leu Glu Leu Ala Val Gln Asn Met Ser Gln Phe Glu Lys
1               5                   10


<210>  100
<211>  12
<212>  PRT
<213>  artificial

<220>
<223>  candidate identity

<400>  100

Asn Ala Leu Ser Glu Ile Asn Leu Gln Glu Ala Arg
1               5                   10


<210>  101
<211>  14
<212>  PRT
<213>  artificial

<220>
<223>  candidate identity
```

<400> 101

```
Glu Phe Phe Ser Ala Phe Asn Gln Leu Tyr Ile Glu Asn Lys
1               5                   10
```

<210> 102
<211> 15
<212> PRT
<213> artificial

<220>
<223> candidate identity

<400> 102

```
Leu Gln Val Ile Asn Ala Phe Ile Asn Ser Gly Gln Asp Pro Lys
1               5                   10                  15
```

<210> 103
<211> 15
<212> PRT
<213> artificial

<220>
<223> candidate identity

<400> 103

```
Gly Gln Gly Thr Asn Glu Tyr Phe Tyr Asn Asp Gln Pro Val Arg
1               5                   10                  15
```

<210> 104
<211> 12
<212> PRT
<213> artificial

<220>
<223> candidate identity

<400> 104

```
Ser Pro Glu Ser Gly Ser Gln Glu Ala Thr Pro Lys
1               5                   10
```

<210> 105
<211> 12
<212> PRT
<213> artificial

<220>
<223> candidate identity

<400> 105

```
Thr Ala Thr Pro Glu Gln Ala Gln Gln Val Cys Lys
1               5                   10
```

<210> 106
<211> 11
<212> PRT
<213> artificial

<220>
<223> candidate identity

<400> 106

Gln Phe Ala Asp Leu Gly Pro Val Tyr Gly Lys
1               5                   10


<210> 107
<211> 12
<212> PRT
<213> artificial

<220>
<223> candidate identity

<400> 107

Ala Phe Glu Ile Asp Gln Ile Leu Asn Ser Leu Lys
1               5                   10


<210> 108
<211> 18
<212> PRT
<213> artificial

<220>
<223> candidate identity

<400> 108

Asn Ser Phe Phe Ala Leu Pro Gln Thr Gly Asn Ser Ile Phe Ser Gly
1               5                   10                  15


Glu Lys


<210> 109
<211> 8
<212> PRT
<213> artificial

<220>
<223> candidate identity

<400> 109

Asn Phe Asp Asp Val Asp Phe Lys
1               5


<210> 110
<211> 13
<212> PRT

<213> artificial

<220>
<223> candidate identity

<400> 110

Asn Glu Ser Ile Ile Ile Ile Val Leu Ser Ser Gly Lys
1               5                   10


<210> 111
<211> 11
<212> PRT
<213> artificial

<220>
<223> candidate identity

<400> 111

Gln Ile Glu Gln Leu Thr Glu Thr Val Gln Lys
1               5                   10


<210> 112
<211> 12
<212> PRT
<213> artificial

<220>
<223> candidate identity

<400> 112

Gln Ala Gly Ala Asp Phe Val Gly Glu Ile Asp Lys
1               5                   10


<210> 113
<211> 12
<212> PRT
<213> artificial

<220>
<223> candidate identity

<400> 113

Ser Ile Val Thr Met Asp Pro Pro Asn Val Gly Arg
1               5                   10


<210> 114
<211> 13
<212> PRT
<213> artificial

<220>
<223> candidate identity

<400> 114

Ser Gly Val Asp Gly Met Val Asp Glu Gly Val Leu Lys
1               5                   10

<210>    115
<211>    13
<212>    PRT
<213>    artificial

<220>
<223>    candidate identity

<400>    115

Ser Ile Thr Phe Gly Val Ser Glu Ala Trp Leu Asn Lys
1               5                   10

<210>    116
<211>    13
<212>    PRT
<213>    artificial

<220>
<223>    candidate identity

<400>    116

Thr Asp Phe Leu Leu Leu Gly Gln Ser Ala Gly Ser Lys
1               5                   10

<210>    117
<211>    17
<212>    PRT
<213>    artificial

<220>
<223>    candidate identity

<400>    117

Thr Glu Asn Thr Gln Gln Ser Glu Ala Pro Gly Thr Asn Thr Gly Asn
1               5                   10                  15

Lys

<210>    118
<211>    13
<212>    PRT
<213>    artificial

<220>
<223>    candidate identity

<400>    118

Thr Glu Val Glu Ser Ser Ile Gln Glu Ile Thr Ser Lys
1               5                   10

```
<210>  119
<211>  12
<212>  PRT
<213>  artificial

<220>
<223>  candidate identity

<400>  119

Thr Asn Gly Gln Ala Asn Pro Asn Val Ser Ile Lys
1               5                   10


<210>  120
<211>  13
<212>  PRT
<213>  artificial

<220>
<223>  candidate identity

<400>  120

Thr Ser Glu Asp Val Leu Gln Ala Ile Asp Phe Leu Lys
1               5                   10


<210>  121
<211>  9
<212>  PRT
<213>  artificial

<220>
<223>  candidate identity

<400>  121

Thr Ser Ile Leu Asn Tyr Asp Ile Lys
1               5


<210>  122
<211>  11
<212>  PRT
<213>  artificial

<220>
<223>  candidate identity

<400>  122

Ser Ala Ala Ala Ala Asn Ile Asn Ile Leu Lys
1               5                   10


<210>  123
<211>  11
<212>  PRT
<213>  artificial

<220>
```

<223> candidate identity

<400> 123

Met Asn Asn Trp Gln Glu Glu Leu Phe Leu Lys
1               5                   10

<210> 124
<211> 8
<212> PRT
<213> artificial

<220>
<223> candidate identity

<400> 124

Met His Gln Ala Gly Ile Pro Arg
1               5

<210> 125
<211> 17
<212> PRT
<213> artificial

<220>
<223> candidate identity

<400> 125

Val Ala Glu Tyr Glu Phe Thr Thr Leu Val Pro Gln Leu Gly Leu Val
1               5                   10                  15

Lys

<210> 126
<211> 9
<212> PRT
<213> artificial

<220>
<223> candidate identity

<400> 126

Ala Leu Glu Pro Asn Ile Val Gly Lys
1               5

<210> 127
<211> 9
<212> PRT
<213> artificial

<220>
<223> candidate identity

<400> 127

```
Ala Leu Asn Gln Ile Pro Ala Phe Lys
1               5
```

```
<210>   128
<211>   14
<212>   PRT
<213>   artificial

<220>
<223>   candidate identity

<400>   128
```

```
Ala Val Ile Thr Val Pro Ala Tyr Phe Asp Asn Ser Gln Arg
1               5                   10
```

```
<210>   129
<211>   9
<212>   PRT
<213>   artificial

<220>
<223>   candidate identity

<400>   129
```

```
Asp Gly Asp Leu Glu Leu Ala Leu Arg
1               5
```

```
<210>   130
<211>   15
<212>   PRT
<213>   artificial

<220>
<223>   candidate identity

<400>   130
```

```
Glu Leu Phe Ile Val Glu Gly Asp Ser Ala Gly Gly Thr Ala Lys
1               5                   10                  15
```

```
<210>   131
<211>   11
<212>   PRT
<213>   artificial

<220>
<223>   candidate identity

<400>   131
```

```
Glu Val Ile Asn Thr Phe Leu Glu Asn Phe Lys
1               5                   10
```

**Description**

**Field of the invention**

The invention relates to a method of determination of identity of an entity from mass spectra. The method is useful in proteomics, metabolomics and its applications in proteomics, metabolomics, genomics and transcriptomics.

**Background Art**

Discovery proteomics contains wealth of rare information, often resistant to reliable interpretation. In shotgun proteomics, discovery-oriented subfield of bottom-up proteomics, proteins are enzymatically cleaved into peptides and the digested samples gradually introduced into an analyser, most commonly into a mass spectrometer using liquid chromatography. In mass spectrometry analysis, typically, in each cycle, masses of intact molecules are analyzed, those of further interest isolated, fragmented and second mass analyses performed on fragments, giving MS/MS spectra. The goal of identification is the peptide producing observed MS/MS spectrum and mapping of peptides to proteins concludes the protein identification task.

Because of immense proteome complexity, number of potential interpretations of each fragment spectrum is enormous. Size apart, even unbiased consideration of all interpretations is of limited use as high spectral homology draws many interpretations equal - a phenomenon often witnessed in peptide sequencing *de novo.* In practice, identification is intentionally biased towards much smaller database of reference peptides, commonly using search engines such as Sequest or X!Tandem. Reference search leaves many spectra uninterpreted, around 75% as recently evaluated on projects in PRIDE repository. Although many unmatched spectra were confidently found to contain peptides with post-translational modifications, majority still remains without interpretation. Several explanations remain open, e.g., splicing alterations, re-arranged or novel genes, however unmatched spectra often exhibit properties of peptides. As human proteomes differ from reference in around ten thousand peptide amino acid sites, some spectra might be naturally attributed to variants and their identification is the aim of the present invention.

The same problems with interpretation of outcomes of analyses of complex samples are encountered in many other fields of chemical, biochemical and biological research dealing with complex mixtures, irrespective of the analytical methods used and irrespective of the structural character of the samples.

**Summary of the invention**

The present invention relates to a method for determination of identity of at least one entity from a mass spectrum of said at least one entity and optionally from additional data from chemical, biochemical or biological analysis of said at least one entity, for each entity comprising the steps of:

a) collecting analytical data from mass spectrum of the entity, and optionally collecting additional analytical data from a chemical, biochemical or biological analysis of the entity,

b) obtaining a plurality of candidate identities of the entity and obtaining the prevalences of said candidate identities of the entity, whereas for each candidate identity it applies that all candidate identities with a higher prevalence are included in the plurality of candidate identities;

c) for each candidate identity of an entity, calculation of its score, said calculation involving at least prevalence of entity, or at least prevalence of entity and agreement with mass spectrum,

d) determining the identity of an entity as the candidate identity with the score closest to the score which would correspond to the true identity of the entity.

The candidate identities selected in step b) comprise candidate identities which are a possible or admissible interpretation of the mass spectrum and optionally of the additional data.

The score calculated in step c) and used for finally determining the identity in step d) may have a form of a numerical value (then in step d), usually the highest value of the score determines the identity which is finally determined to be the correct one for the analyzed entity), or another form, such as an interval of numbers, a non-numerical entity, entities with established order, a number with probabilistic interpretation. The skilled person will appreciate that when a form of the score is selected, also the score which would correspond to the true identity of the entity (the ideal score) is selected or determined by the form of the score or by its calculation. E.g., for a number with probabilistic interpretation, 100% probability (or value 1) corresponds to the true identity of the entity. The "true identity" is meant as the real identity of the entity, which is however unknown at the beginning of the process.

Preferably, in the step c), the calculation involves calculating maximal probability of candidate identity. The maximal probability may be the score, or it may be a variable in the calculation of the score.

Preferably, in the step c), the calculation involves calculating probability of candidate identity. The probability may be the score, or it may be a variable in the calculation of the score.

Preferably, in the step c), the calculation involves calculating probability of candidate identity using Bayes' Theorem.

In one preferred embodiment, in the step b) the value of prevalence is calculated based on at least one of population frequency of said entity, probability of modification of said entity in the environment, probability of modification of said entity during the analysis step.

Preferably, in the steps b) and c), the value of prevalence is expressed as prior probability or as prior-like probability.

In a preferred embodiment, in the step d) the determination of identity comprises evaluating whether multiple forms of isotopically labeled peptides were present.

Preferably, the entity is selected from a molecule having the molecular weight of up to 2000 mol/g, a peptide, a protein, a lipid, a nucleic acid, a metabolite.

Preferably, the entity is a peptide, and the method used to obtain the mass spectrum is tandem mass spectrometry (also referred to as MS/MS).

In preferred embodiments which are further referred to as comprising "enumeration", in step b), the obtaining of the candidate entities and/or of prevalence of the candidate identities comprises enumeration which comprises the steps of:

b.a) selecting initial candidate identities with initial prevalences;
b.b) transferring said initial candidate identities into a base of candidate identities;
b.c) producing new candidate identities by application of events to said base of candidate identities, and incorporating said new candidate identities into said base of candidate identity identities and continuing said producing unless a limiting condition is met;
b.d) transforming the base of candidate identities obtained in step b.c) into candidate identities with associated prevalence.

In the enumeration embodiments, preferably said candidate identities are peptides; said prevalence is expressed as a prior-like probability; said initial entities are N-terminally-cleaved linear subsequences of reference proteins; said applicable events comprise modification, substitution and cleavage; said limiting condition is minimal prior-like probability of given form of peptide.

In the enumeration embodiments, preferably said candidate identities are proteins; said prevalence is expressed as a prior-like probability; said initial entities are reference exon-based protein models; said applicable events comprise exon exclusion and exon inclusion; said limiting condition is minimal prior-like probability of exon-based model; said transformation of entities into hypotheses is concatenation of exons into protein-coding sequence and translation in silico.

The method of the present invention has a number of potential utilizations, which may involve additional steps upstream or downstream, or may involve the utilization of the determined identity of one or more entities by the method of the present invention in known methods.

The method of the present invention, wherein the entities are proteins, wherein the step of obtaining the candidate identities of an entity in step b) includes database search in database of peptide variants, may be used for identification of mutant and polymorphic proteins from mass spectra of proteome, with alterations already observed globally on nucleotide level.

The method of the present invention, wherein the entities are peptides, further comprising the steps of: e) matching of entities determined as polymorphic peptides to database of origins, may be used for determination of identity on the basis of variability of known prevalence, in particular for authentication of cell lines or identification of an individual from mass spectra of proteome.

The method of the present invention, wherein the entities are non-host peptides, wherein in the step b) the prevalence is expressed as prior or prior-like probability and wherein the prevalence of non-host peptides is scaled down according to prevalence of non-host organism,

may be used for identification of non-host organism of known prevalence from mass spectra of proteome of host organism.

The method of the present invention, wherein the entities are non-host peptides, wherein in the step b) in obtaining the candidate identities, peptides uniquely mapping to non-host organism are added to enumerated peptides of host organism and prevalence of non-host peptides is lower than of any host peptide, may be used for identification of non-host organism of unknown prevalence from mass spectra of proteome of host organism.

The method of the present invention, wherein the entities are donor peptides, wherein in the step b) the prevalence of donor peptides is scaled according to their prevalence among recipient peptides, may be used for identification of proteins originating from grafted tissue in allograft or xenograft.

The method of the present invention, wherein the entities are peptides, the method further comprising the step of: e) selecting somatic mutant peptides attributable to tumour, may be used for identification of presence of a tumour and estimation of its stage from mass spectra of circulating proteins.

The method of the present invention, wherein the entities are peptides, the method further comprising the step of: e) selection and quantification of polymorphic peptides attributable to donor, may be used for monitoring organ transplantation and early detection of transplant rejection from mass spectra of blood plasma or serum of recipient.

The method of the present invention, wherein the entities are peptides, said method further comprising the step of: e) calculating significance of match between two individuals based on polymorphic peptides, may be used for determination of presence of genetic relationship between two individuals from measured mass spectra of proteome.

Furthermore, the invention encompasses a data processing system comprising means for carrying out the steps of the method of any one of the preceding claims.

The invention also encompasses a computer program comprising instructions which, when the program is executed by a computer, cause the computer to carry out the steps of the method of any one of the preceding claims.

Yet furthermore, the invention encompasses a computer-readable medium comprising instructions which, when executed by a computer, cause the computer to carry out the steps of the method of any one of the preceding claims.

## Brief description of the drawings

In the drawings which schematically represent the method or its steps and sub-steps, lines with arrows refer to direct or indirect connection between individual units. Dotted lines with arrows correspond, in general, to alternative embodiments. Alternative embodiments are further indicated with addition of alphabetical letters grouping particular alternative embodiment. Reference numbers of subunits within units are formed as a concatenation of the reference number of the main unit, period and reference number of the subunit. Units depicted on drawings are assumed to be either standalone, or part of some larger units. Dotted outlines of blocks correspond to steps.

Figure 1 is a schematic representation of incorporation of prevalence models into identification methods.

Figure 2 is a schematic representation of incorporation of prevalence model for reevaluation.

Figure 3 is a schematic representation of incorporation of prevalence model within identification system.

Figure 4 is a schematic representation of incorporation of prevalence model for influencing selection of candidate identities.

Figure 5 is a schematic representation of enumeration.

Figure 6 is a schematic representation of enumeration of peptides in shotgun proteomics.

Figure 7 illustrates use of variants for identification of origin.

Figure 8 is a schematic representation for evaluation of correspondence between entities.

Figure 9 illustrates an MS/MS spectrum of particular precursor measured using tandem mass spectrometry.

Figure 10 illustrates behaviour of particular agreement model in shotgun proteomics.

Figure 11 illustrates behaviour of particular agreement model of true interpretations in shotgun proteomics.

Figure 12 illustrates behaviour of particular agreement model of random interpretations in shotgun proteomics.

Figure 13 is an example of distribution of precursor mass difference for true matches.

Figure 14 is an example of experimental distributions of retention time at given theoretical retention time.

Figure 15 shows a selection of true matches based on extreme behaviour of retention time.

Figure 16 shows a distribution of differences of theoretical and experimental isotopic distributions.

Figure 17 shows an example of combination of precursor mass difference and retention time into one value.

Figure 18 shows the power of filtering when precursor mass difference, isotopic distribution difference, retention time and protein evidence are combined into single criterium.

Figure 19 is a schematic representation of particular example of incorporation of prevalence model in shotgun proteomics.

Figure 20 shows likely incompleteness of exome sequencing data for areas of low sequencing coverage.

Figure 21 illustrates the family structure for calculation of correspondence.

Figure 22 illustrates the behaviour of coverage of reference proteins in pairwise comparison.

Figure 23 illustrates calculation of at least as good match at random between family members.

Figure 24 illustrates results of identification of tumour-specific circulating proteins.

Figure 25 illustrates identification of human mutant biomarkers in murine xenograft models.

Figure 26 illustrates identification of microbial peptides and demonstrates practical use for diagnostics of microbial pathogens in human and animal materials.

Figure 27 is a schematic representation of enumeration of splice variants in proteomics.

Figure 28 illustrates correspondence of tumour size versus proportion of somatic variants among identified peptides.

## Detailed description of the invention

"Entity" herein refers to a chemical or biological entity, such as a molecule, substance or organelle. In particular, entity may be selected from a substance, a compound, a lipid, a metabolite, a peptide, a protein and a nucleic acid.

"Prevalence" herein refers to frequency of occurrence of an entity. The frequency of occurrence of an entity refers to its frequency of occurrence in the nature, or in a specific part of the nature which was the source of the measured sample, such as organism, part of organism, specific environment, etc. Prevalence can be expressed in relative terms, e.g., entity A being more prevalent than entity B, or in absolute terms, such as percentages or amounts of the entity per unit of the sample or of the part of the nature. Prevalence also includes prior probabilities of entities. Prevalence also includes relative probabilistic terms, referred here as prior-like probabilities, wherein the relative differences between entities are the same as for prior probabilities of entities.

"Identity of an entity" herein refers to the determination of structural information about the entity, such as its chemical structure, sequence of amino acids or nucleotides. The structural information may refer to assigning a known structure to the entity, or determining its structure or part of its structure even though previously unknown.

"Candidate identity" herein refers to possible or admissible explanation (or interpretation) of the observed mass spectra and optionally additional chemical or biological data.

"Enumeration"herein refers to method of construction of candidate identities and their prevalence which is based on initial candidate entities and events for their combination. Such events include modifications of the initial entities which may have occurred.

"Score" is a value calculated for each candidate identity. The score may have a form of a numerical value, vector or array of numerical values, interval of numbers, non-numerical entity, entities with established order. Score also includes number with probabilistic interpretation, for example, probability of correctness, p-value, E-value, q-value, maximal probability and their intervals. The skilled person will appreciate that when determining the form of the score, its value which would correspond to the true identity of the entity is also determined. E.g., for a score corresponding to probability, the value corresponding to the true identity of the entity is 1 or 100%.

"Mass spectrum" refers to mass spectrum (MS) obtained by introducing the entity into a mass spectrometer and performing the mass spectrum measurement, or to MS/MS spectrum. Analytical data from mass spectrum are typically the data about the fragment peaks shown in the spectrum (m/z values, intensities). Additional criteria from the mass spectrum may also be used, such as precursor mass difference, isotopic distribution difference, protein evidence.

"Chemical, physical, biochemical or biological analysis" include any analytical methods allowing to obtain data useful for the determination of identity of the entity. Such methods include spectroscopic analytical methods such as NMR spectroscopy, X-ray diffraction spectrometry, IR spectroscopy; immunochemical methods; optical observation methods; methods relying on interaction with further agents, such as antibodies, labels.

"Explanation" and "interpretation" are herein used to designate the assignment of the identity of at least one entity to an analytical method outcome, i.e. to a mass spectrum and optionally additional data.

The present invention describes a method of determination of identity of an entity based on their mass spectrum data, and optionally additional data from other analytical methods, said method utilizing prevalence data and prevalence or probabilistic calculations. Use of prevalence provides additional layer of discrimination and thus helps in resolution of otherwise indistinguishable situations. For instance, it is often the case that there are many explanations which agree equally well with an observed mass spectrum and additional data. The use of prevalence models might enable distinguishment between these explanations if one explanation is much more prevalent than the rest. In effect, the utilization of prevalence reduces the complexity of the identification task.

If the way the prevalence is modelled, which is herein referred to as a "prevalence model", exhibits desirable properties (e.g., the prevalence is expressed in relative probabilistic terms), the candidate interpretations might be often assigned a probability of correctness, or a maximal probability of correctness. Probability of correctness of an explanation has in turn the advantage of being usable in real-life scenarios as it enables long-term modelling of decision-making processes. Similarly, maximal probability of correctness provides strong grounds on which to rule out candidate explanations with direct real-life applicability. This might be shown in contrast to statistical significance of agreement (e.g., p-value or E-value), which does not possess such quality and even highly significant agreement might be often assigned to incorrect interpretations. This behaviour is practically impossible for a properly derived probability of correctness and thus gives practically much more desirable guarantees. The utilization of prevalence models especially helps in reliable identification of rare events. In case the prevalence of candidate explanations varies significantly (e.g., spanning multiple orders of magnitude as is the case in bottom-up proteomics), incorporation of prevalence might be necessary to obtain reliable results.

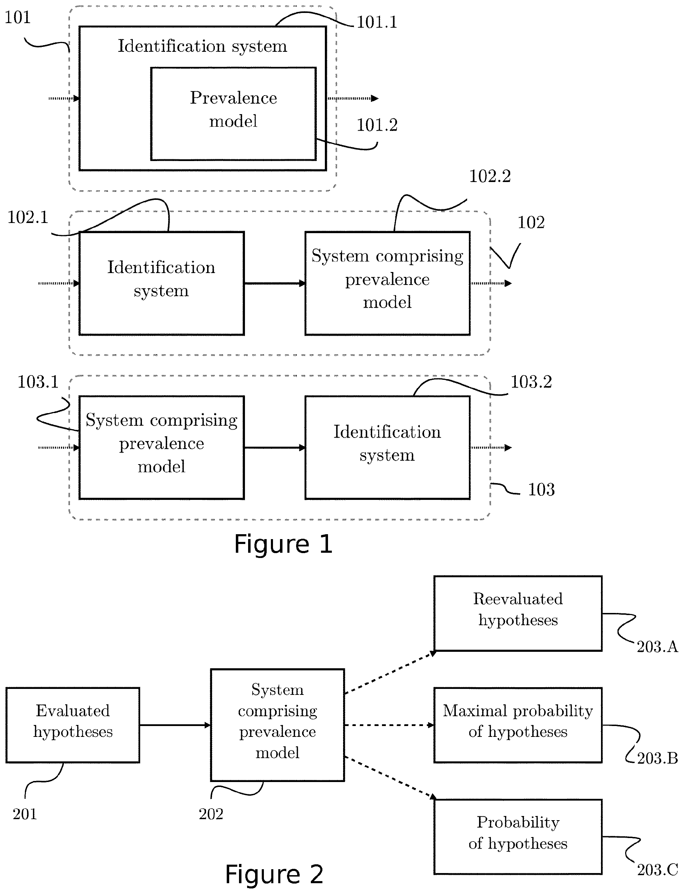

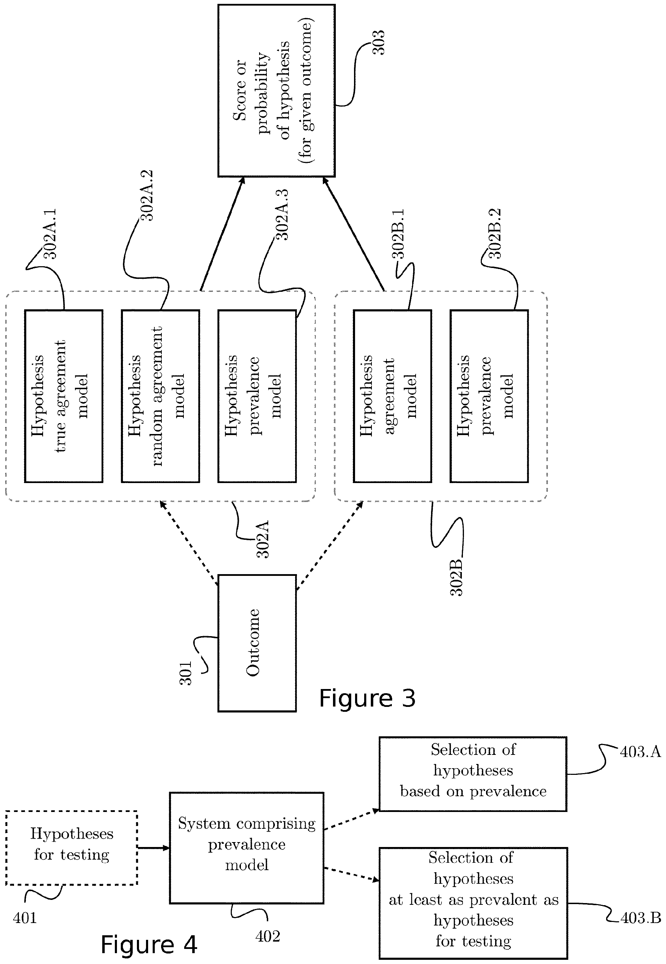

Fig. 1 represents several basic configurations of incorporation of prevalence model into identification system. In some embodiments 101, the prevalence model 101.2 is integrated into the identification system 101.1. Such incorporation is preferable for derivation of probability of correctness of candidate identity. More specific embodiments are illustrated on Fig. 3.

In other embodiments 102, the identification system 102.1 is separate from a system 102.2 comprising prevalence model, in this configuration the system 102.2 comprising prevalence model process results from said identification system 102.1. Such embodiments are usable for instance to derive maximal probability of candidate identity or probability of candidate identity. More specific embodiments of this kind are further illustrated on Fig. 2.

Yet other embodiments 103 comprise an identification system 103.2 and a system 103.1 comprising prevalence model in which the identification system 103.2 works with the selection of candidate identities influenced by the prevalence model 103.1. Such embodiments can be used to preselect candidate identities in a way which improves the behavior of the identification system. More specific embodiments of this kind are illustrated on Fig. 4.

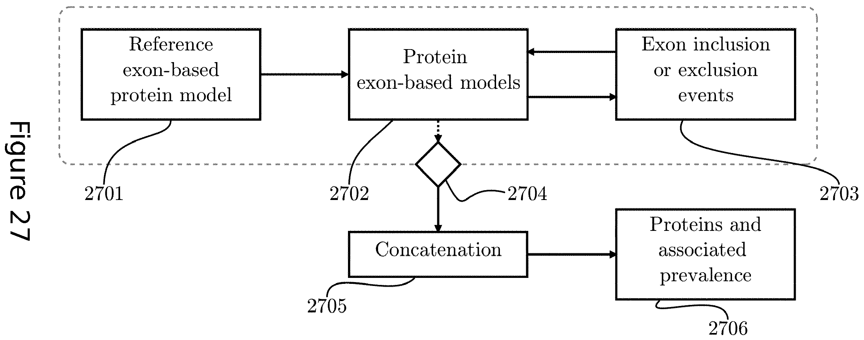

Fig. 2 represents incorporation of a prevalence model for reevaluation of candidate identities. In this embodiment, evaluated candidate identities 201 pass through a system comprising the prevalence model 202. There are various possible alternatives. In an alternative embodiment 203.A, candidate identities are evaluated utilizing information from prevalence model. In such reevaluation, for instance, a new information can be added; for example the number of candidate identities which are at least as prevalent as the hypothesis and have the same agreement with the observed data as the candidate identity. In another embodiment 203.B, the candidate identities are assigned the maximal probability of their correctness. Specific embodiment of this kind in shotgun proteomics is illustrated on Fig. 27. In some embodiments 203.C, the candidate identities are assigned the probability of their correctness.

Fig. 3 represents embodiments used for determination of identity in identification wherein the prevalence model is integrated within the identification system. This configuration is in general suitable for scoring and derivation of probability of correctness of the candidate identity. In some embodiments, the identification system 302A comprises a true agreement model 302A.1, a random agreement model 302A.2 and a prevalence model 302A.3. Such configuration is particularly suitable for derivation of probability using Bayes' Theorem. In some embodiments, the identification system 302B comprises an agreement model 302B.1 and a prevalence model 302B.2 to obtain score or probability of candidate identity 303.

Fig. 4 represents incorporation of prevalence model to influence selection of tested candidate identities. In some embodiments 403.A, the selection of candidate identities is influenced based on their prevalence. An example in shotgun proteomics is selection of peptides more prevalent than peptides with some modification (for example, methylation), or amino acid substitution, or peptides more prevalent than peptides resulting from splicing alteration. An example in top-down proteomics is selection of proteins similarly prevalent as non-modified proteins.

In alternative embodiments 403.B, the selected candidate identities are at least as prevalent as the candidate identities accepted initially for testing (hypotheses 401). An example in bottom-up proteomics is when candidate identities 401 for testing correspond to variant peptides, and candidate identities which are at least as prevalent as the variant peptides 402 are selected in step 403.B (based on particular assumptions over prevalence of individual candidate identities).

The first step of the present invention comprises collecting analytical data. The methods for collecting analytical data, in particular mass spectrometry data, are well known to those skilled in the art. For example, in shotgun proteomics, the sample preparation protocols are well established and in general process samples into mixture of proteolytic peptides; see for instance an article comparing three protocols FASP, SP3 and iST (Sielaff et al. (2017): Journal of Proteome Research, 16(11): 4060-4072). Identification of the substances starts with their physical separation using liquid chromatography, coupled with mass spectrometer. Substances eluting at a particular time (retention time) enter mass spectrometer, undergo ionization and their masses are measured, giving a precursor spectrum. In data dependent acquisition, after each such measured precursor spectrum, masses of several most abundant precursors are selected, the ions are isolated, fragmented and MS/MS spectra (also fragment, or product spectra) acquired. These fragment spectra are of interest because calculation of the score may comprise evaluation of the agreement of theoretical spectra of candidate identities and the observed spectra of the entities.

In the second step of the method of the invention, candidate identities of the analyzed entity are obtained. This step can be performed obtained in multiple ways.

In the usual scenario, candidate identities are obtained through a database search of entities for the given samples. For example, the search may be for peptides or nucleic acids or lipids or compounds or metabolites for given analyzed organism. Often, candidate identities are obtained through reference database search containing reference entities (e.g., peptides) for the analyzed organism. Examples of such databases are UniProt and ENSEMBL. If the analyzed entities are proteins or peptides, then proteins from these databases are *in silico* digested with a protease used in the experiment. As reference proteolytic peptides are of highest prevalence, they are self-contained in the sense that all more prevalent peptides (than the peptide of the lowest prevalence) are considered as well. However, if some modifications of the reference entities are considered, care must be taken such that all modifications at least as prevalent as the modification of lowest prevalence are considered as well.

In some embodiments, the candidate identities may be obtained using enumeration of candidate identities.

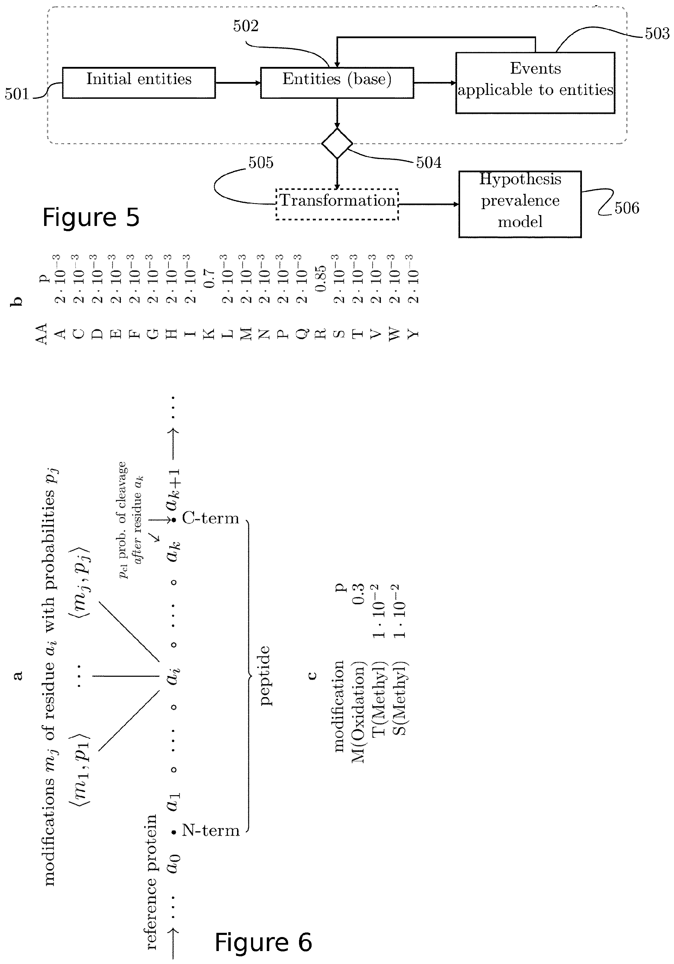

Fig. 5 schematically shows the general process of enumeration, in which initial entities and events applicable to entities (e.g., chemical modifications occurring in the nature) are used for construction of prevalence model. In the beginning, initial entities 501 with associated prevalence are transferred to the base 502 of entities. The base 502 of entities is a part of a cycle, unlike initial entities. Entities from the base 502 are subjected to events 503 (in silico) which create additional entities, which are incorporated into the base 502. This continues until a pre-defined criterion 504 is met. Then the process stops, entities in the base 502 are optionally transformed in step 505 (if necessary) into the final form which then constitutes the prevalence model 506. This process has an important advantage, when coupled with prevalence: for each candidate identity $e$ enumerated, all candidate identities which are at least as prevalent as said candidate identity $e$ are enumerated as well.

Example of embodiments related to shotgun proteomics, i.e., wherein the analyzed entities are proteins, is used to describe some preferred features of the enumeration. The enumeration shown in Fig. 6a is used for assignment of prevalence to reference peptides, variant and modified peptides of varying cleavage specificity. The enumeration is performed over each reference protein independently and the behaviour for particular reference protein is described as follows. As initial candidate identities for a reference protein, all N-terminally cleaved sequences of said protein are used. The prevalence of these candidate identities depends on the probability of cleavage after residue just before the cleavage point (herein, $a_0$, in Fig. 6). For example, in case of tryptic digestion, the initial prevalence will be usually large in case of lysine and arginine. In this example, if it is at N-terminus of the protein, the initial prevalence equals 1 (no cleavage needed). These initial candidate identities are transferred to the base of candidate identities. The events applicable to candidate identities are as follows: extension, modification and cleavage. Extension refers to the event of incorporation of next residue in reference amino acid chain and the probability of extension is derived as a complementary event of cleaving. Cleavage is modelled as cleaving after a specific amino acid and each candidate identity needs an exactly one cleavage to become a fully formed candidate identity (such cleavage does not need to happen at the C-terminus of the protein). Modifications $(m_1,...,m_j)$ with respective prior-like probabilities $(p_1,...,p_j)$ are applicable to each amino acid. Further, statistical independence of events is assumed, which enables assignment of prevalence in the form of prior-like probability to every peptide by multiplication of prior-like probabilities of events. The process continues until the minimal prior-like probability is met, which constitutes the stopping criterion. Herein, the entities themselves were candidate identities, therefore there is no need for any transformation step, and thus the base of entities is then taken for the prevalence model.

In some embodiments, prior-like probabilities are involved in the prevalence models and/or in the calculation of the score. Prior-like probabilities are also referred to in literature as relative probabilities. For prior-like probabilities, the relative proportions between individual prior-like probabilities are the same as for prior probabilities. Thus the following holds: suppose selection of large number $n$ of outcomes (e.g., MS/MS spectra) and their interpretation by candidate identities $q$ and $r$ with prior-like probabilities $\mathrm{Pr}_q$ and $\mathrm{Pr}_r$, respectively. The proportion of correct interpretations by candidate identity $q$ as compared to $r$ is then

$$\mathrm{Pr}_q \cdot \mathrm{Pr}_r^{-1}. \qquad (1)$$

Thus prior-like probabilities preserve relative differences between prior probabilities.

As an example of preferred features and prior-like probabilities, the establishment of prior-like probabilities in proteomics is described. Prior-like probabilities can be derived from experimental data under these assumptions: the measured data represent the whole population; and the subset of data which is assumed to be correctly interpreted does not change the distribution.

Function $\Gamma$ is used from measurements to peptides

$$\Gamma : \mathbb{O} \to \mathbb{H} \qquad (2)$$

which gives for each measurement $s$ (MS/MS spectrum) a true interpretation $q$ (peptide) of the spectrum (assuming that there is exactly one true interpretation).

For including the modifications of the peptides, the following is used: Suppose peptide $q$ represented as a tuple

$$q = \langle a_1 \oplus m_1, \ldots, a_n \oplus m_n \rangle, \qquad (3)$$

where each $a_i$ is a coded amino acid residue and each $m_i$ is a modification applicable to the residue $a_i$. The set of applicable modifications to $a_i$ is denoted as $\Phi(a_i)$ and for technical brevity, existence of empty modification is considered.

Denoting by uppercase $Q$ the set of all modified forms of peptide $q$, the proportion $r_q$ of a particular form of peptide $q$ to all its forms $Q$ is expressed as

$$r_q = \frac{P(q = \Gamma(s))}{\sum_{q \in Q} P(q = \Gamma(s))}. \tag{4}$$

In practice, there is not enough data to model each peptide separately. It is assumed that their behaviour is independent of peptide sequence and depends only on the modification $m$ itself. For the purpose, peptides can be considered with exactly one residue to which $m$ is applicable, denoting such set as $H_m$. Denote

$$\mathbb{H}_m^+ \subseteq \mathbb{H}_m \tag{5}$$

the peptides with the modification. Then, for a particular modification $m$, the

$$r_m = \frac{P(\Gamma(s) \in \mathbb{H}_m^+)}{P(\Gamma(s) \in \mathbb{H}_m)} \tag{6}$$

is equal to (4) for each peptide $q$ and can be calculated from larger set of data.

Furthermore, the approach can be extended to account for peptides with varying number of modifiable residues. Such extension behaves in the same way on peptides with exactly one residue and enables the utilization of the whole set of interpretations. Specifically, the proportion for modification $m$ on residue $a$ is derived as the total number of $a$ residues modified with $m$ to the total number of $a$ residues with any applicable modification (also the empty one). Suppose

$$n(a \oplus m) \tag{7}$$

refers to the number of $a$ residues with modification $m$. Then the proportion $r_m$ can be derived as

$$r_m = \frac{n(a \oplus m)}{\sum_{m \in \Phi(a)} n(a \oplus m)} \tag{8}$$

As another example, probabilities of DNA/RNA substitution are derived. Derivation is analogous as for modifications but with the following difference in the modelling approach. Due to the low ratio of substitutions in the data, the substitution event is modelled in an aggregated manner (independent of the residue). Specifically, the proportion r of all altered residues to all residues is obtained

$$r = \frac{\sum_a n(\hat{a})}{\sum_a n(a)}, \tag{9}$$

and interpret it as a probability of amino acid substitution.

As yet another example, for derivation of cleavage probabilities (after particular amino acid) peptides with missed cleavages and semi-specific cleavage (specific at N-terminus and not specific at C-terminus) were utilized. Denoting $n^{\text{cleavage}}(a)$ as the number of residues $a$ followed by cleavage, and $n(a)$ as the total number of residues $a$, the proportion $r_a$ of cleavages after residue $a$ is

$$r_a = \frac{n^{\text{cleavage}}(a)}{n(a)}. \tag{10}$$

As yet another example, calculation of prevalence of peptides in allografts or xenografts is shown, for identification of peptides originating from grafted tissue in tumour xenograft model. In this case, the prevalence of peptides from different organisms (donor and recipient) is different and needs to be taken into account. Often the recipient would be an animal model and the donor would be a human; peptides from the animal are expected to be more prevalent than those of the human, for instance to monitor tissue/organ grafting or rejection or identification of donor's peptides in the recipient. Alternatively, the recipient can be a patient undergoing tissue transplantation and the donor can be a tissue/organ donor. In the following, it is shown how to estimate the difference in the prevalence of donor and recipient peptides.

The relative difference in the prevalences of donor peptides to recipient peptides can be estimated through derivation of the origin of homologous peptides of the donor and the recipient. Suppose a homologous peptide attributable to both donor and recipient was identified. The interest is in knowing whether the peptide is from the donor or the recipient. For this purpose, protein evidence (of donor proteins and of recipient proteins) of a given peptide can be used which provides the evidence of the origin of the peptide. The proportion $p$ is estimated as the proportion of the homologous peptides with the donor protein evidence as compared to those with the recipient protein evidence. In the construction of the protein evidence, the protein evidence is restricted to heterologous peptides only. In another approach, the proportion $p$ is estimated as the ratio of detected heterologous peptides. Both approaches can be used when there is a limited homology between donor and recipient, which is often the case in xenografts. In allografts, the proportion can be set equal. From a practical perspective, the relative difference between prevalence of donor and recipient peptides is rather small; for instance the number of the donor peptides is in the order of tens of percent of those from recipient. This is important to note as it simplifies identification of donor peptides as there is no other organism (other than donor) expected to be of higher prevalence than that of recipient.

As another option, determination of prevalence of peptides of non-host organisms is described. The situations when identification of non-host organism is of interest include for example detection of microbial presence in an organism, for example for diagnosis of microbial infection.

In some situations, prevalence is known. In general, it is necessary to take the prevalence (or its estimate) of the non-host organism into account. The situation is partially similar to allografts or xenografts, however with the difference that the prevalence of peptides of the non-host organism is generally lower than that of a grafted tissue and non-host peptides are phylogenetically more distant. This has some consequences, notably that all non-host organisms of higher prevalence need to be considered as well (among other at least as prevalent peptides). If the estimate of prevalence ($p_o$) of the non-host organism $o$ is known and also the prevalences of all organisms $q$ at least as prevalent as the organism $o$, $p_o \leq p_q$, then the prevalence model can be easily configured as follows. The prevalence should be expressed in prior or prior-like probabilities and then the prevalence of non-host peptides of the organism $o$ is multiplied with the value of prevalence $p_o$.

Often, the prevalence of the non-host organism is unknown, which complicates the identification task. It is nevertheless possible to increase the specificity of identification, if it can be ascertained that all at least as prevalent organisms are considered as well. In case of unknown prevalence, one possible resolution is to consider all known organisms. This is because it is rather unlikely that organisms which were not described yet are more prevalent than the one we are interested in identification.

In the strictest scenario, all peptides of the non-host organism of interest are assumed of the lowest prevalence among all peptides of all considered organisms. An example of realization of such scenario is to enumerate all organisms with a limiting prior-like probability $r$ (e.g., and estimated prior-like probability, such as $r = 4 \cdot 10^{-6}$) and further scale down prevalences of non-host organisms. In particular, the prevalence of the host remains identical, but the prevalence of all non-host organisms except the one of interest is multiplied by r, and the prevalence of the non-host organism of interest is multiplied by $r^2$. In such case, the non-host peptides of interest are of strictly the lowest prevalence. As the prior-like probabilities are just very roughly established, it is preferable to calculate the maximal probability $P_{max}$ instead of the probability; furthermore in the calculation of $P_{max}$, only the relative order of prior-like probabilities is preferred to be assumed. This scenario requires enumeration of peptides for all known organisms. In such case, the database for $r \approx 4 \cdot 10^{-6}$ will likely have thousands of terabytes.

A more preferred approach to the problem of identifying organisms of unknown prevalence was developed. The computational aspect can be significantly simplified under the following assumptions: i. if a peptide of the non-host organism was measured, it was a fully specific (e.g., tryptic) reference peptide, and ii. all non-host organisms are of equal prevalence which is lower than that of any enumerated peptide of the host. Under these circumstances, the peptides exclusively attributable to the non-host organism of interest can be preselected and added to obtained peptides in enumeration of host.

The third step of the method of the invention, the score is calculated for each candidate entity.

First, agreement metrics used for observed and theoretical spectra is described. Various metrics of agreement between observed and theoretical spectra exist, such as simple number of matching peaks consisting of singly charged ions (b, y ions for CID and HCD). The matching of peaks occurs for prespecified fragment mass tolerance depending on instrumentation used (e.g., 0.3 Th for linear ion trap). The skilled person is aware of the available options and is capable of choosing the suitable one. Derivation of maximal probability of candidate identity can be based on adequacy of the agreement model. The following corresponds to the assertion that a higher agreement of predicted and observed spectrum leads to an increase in the probability of interpretation being correct. Thus for every spectrum $o \in O$, given

two agreements

$$\alpha \le \beta \qquad (11)$$

it is assumed that

$$P\big(p = \Gamma(o) \mid \Phi(p,o) = \alpha\big) \le P\big(q = \Gamma(o) \mid \Phi(q,o) = \beta\big) \qquad (12)$$

where

$$\Gamma : \mathbb{O} \to \mathbb{H} \qquad (13)$$

is a function giving correct interpretation for a spectrum and

$$\Phi : \mathbb{H} \times \mathbb{O} \to \mathbb{X} \qquad (14)$$

an agreement model (for some set X, on which there is order). Furthermore, it is assumed that the same agreement for different candidate identities gives an equal probability of being the correct interpretation from the viewpoint of agreement:

$$P\big(p = \Gamma(o) \mid \Phi(p,o) = \alpha\big) = P\big(q = \Gamma(o) \mid \Phi(q,o) = \alpha\big) \qquad (15)$$

For the candidate interpretation $q$ of $o$, with the prior-like probability $Pr_q$, there is a need to obtain a set R of all at least as good interpretations. R is of the following form

$$R = \big\{ \langle p, Pr_p \rangle \mid p \in \mathbb{H} \text{ and } Pr_p \ge Pr_q \big\} \qquad (16)$$

wherein $p$ is a candidate identity and $Pr_p$ its prior-like (or prior) probability. Thus for the establishment of $P_{max}$, only candidate identities which are a priori equal or more likely need to be considered.

If the correct order of prior-like probabilities is considered, then the maximal probability $P_{max}$ of $q$ is inversely proportional to the number of the at least as good interpretations, thus

$$P \le |R|^{-1} = P_{max}. \qquad (17)$$

If numerical aspects of prior-like probabilities are assumed, then $P_{max}$ is the proportion of $Pr_q$ among all the at least as good interpretations, thus:

$$P \le \frac{Pr_q}{\sum_{\langle p, Pr_p \rangle \in R} Pr_p} = P_{max} \le |R|^{-1}. \qquad (18)$$

The value of $P_{max}$ is independent of search space size.
Preferred methods for derivation of the probability of candidate identity, with prior-like (or prior) probabilities, are described herein. The probability that a particular candidate identity $h$ is the true candidate identity $h = \Gamma(o)$ of the spectrum $o$, given its agreement $d = \Phi(h,o)$ is:

$$P\big(h = \Gamma(o) \mid \Phi(h,o) = d\big) \qquad (19)$$

Such probability can be derived, for example, using Bayes' Theorem, wherein (19) equals to

$$\frac{P\Big(\Phi(h,o) = d \,\Big|\, h = \Gamma(o)\Big) \cdot P\Big(h = \Gamma(o)\Big)}{P\Big(\Phi(h,o) = d\Big)}.$$

$$(20)$$

The equation (20) can be easily changed to incorporate prior-like probabilities, instead of prior probabilities. In case of prior probabilities, the following holds for each $o \in O$, if exhaustively all candidate identities $h \in H$ are considered:

$$\sum_{h \in \mathbb{H}} P\Big(h = \Gamma(o)\Big) = 1$$

$$(21)$$

In case of prior-like probabilities, the sum (21) might be different, but because prior-like probabilities preserve relative differences, they can be always normalized by rescaling.

Prior-like probabilities are easier to establish, however it might be not clear how they should be rescaled. If candidate identities are selected, such that the true candidate identity is among them, then prior-like probabilities can be rescaled to sum to 1 and then are equivalent to prior probabilities.

If the true identity of the analyzed entity is not within the given candidate identities

$$\mathbb{H}_0 \subsetneq \mathbb{H}$$

$$(22)$$

and therefore

$$\sum_{h \in \mathbb{H}_0} P\Big(h = \Gamma(o)\Big) = c < 1.$$

$$(23)$$

The variable $c$ in (23) then corresponds to the probability that the true identity of the analyzed entity is within the selected candidate identities $H_0$. Then, prior-like probabilities of selected candidate identities $H_0$ can be rescaled (their sum) to $c$ and will be equal to prior probabilities.

For instance, in shotgun proteomics, not all candidate interpretations of the spectrum are considered and therefore the true interpretation might be not among them. However, around 25% of spectra are usually correctly interpreted in standard bottom-up proteomics experiment. Thus the value of $c \geq 0.25$ and $c \leq 1$. This also limits the possible range of prior probabilities in shotgun proteomics and in turn the range of probability of correctness.

In mass spectrometry, multiple additional (supporting) criteria, e.g., precursor mass difference, can be directly used in identification. Furthermore, these criteria are useful for identification of rare events, for instance variant peptides. For practical purposes, it is often desirable for these criteria to have statistical interpretation for simplification of making decisions. In particular, the probability was modelled that the true interpretation of a spectrum has a specific additional/supporting criterion at least as extreme as was observed. This in effect enables removal of interpretations.

An example of selection of true interpretations in proteomics field in experiments over a particular organism (for example, human) is mentioned here. There is no need for the interpretations to be correct, rather that the distribution of these criteria to be same as for correct interpretations. Therefore some degree of incorrect results (e.g., 10%) will very likely not invalidate the results. It is assumed that all tryptic reference peptides of expected organism (e.g., human) with sufficient spectral significance (herein, E-Value of 0.1 in X!Tandem in the reference peptide database search) selects suitable interpretations for modelling of supporting criteria.

In some embodiments, precursor mass difference is used as an additional criterion. Distribution of differences between observed and calculated mass of peptide for true interpretations can be readily calculated. Further, association of probabilistic interpretation to differences enables their direct use in identification.

It is assumed that the distribution $D$ of $n$ differences between observed and calculated precursor mass for correct spectral interpretations matches

$$D = \langle d_1, \ldots, d_n \rangle. \qquad (24)$$

The number $n$ is often rather large (order of thousands, or tens of thousands) for a particular sample, or even for a single run on modern instruments (such as Orbitrap). Therefore it is not even necessary to model the distribution and thus it is possible to work directly with data, e.g., through percentiles. For probabilistic interpretation of the difference $d$, $D$ is utilized to calculate $p_d$ as proportion of true matches having at least as extreme difference as is $d$. Thus for difference $d$, we are interested in

$$p_d = \frac{|\{e \in D \mid e \geq d\}|}{|D|}. \qquad (25)$$

Thus if it is unlikely (e.g., $p_d$ at most 0.01) for true match to have at least as extreme precursor mass difference, it provides probabilistic grounds for removal of interpretation.

Mass spectrometry is in modern settings coupled to liquid chromatography which enables utilization of the predicted and observed retention time, similarly as the precursor mass difference. In practice, it is also beneficial to have a statistical interpretation of the difference between those two. In the simplest cases, the retention time difference can be modelled exactly as a precursor mass difference explained above. The prediction of the retention time can be done, for example, via BioLCCC (Liquid Chromatography of Biomacromolecules at Limiting Conditions; http://theorchromo.ru/).

It is preferred, however, to model the behaviour more locally, especially because the observed retention time often shows a non-linear behaviour as related to predicted time. The modelling can be performed for each predicted time $t_i$ separately and for each $t_i$ construct the distribution $D_i$. Each $D_i$ consists of an experimental times $e_j$ (experimental counterparts of $t_j$, $t_j$ being neighbors of $t_i$). Each $D_i$ contains $2 \cdot w$ neighbors, where $2 \cdot w$ is the window size (the preferred size is 500):

$$D_i = \langle e_{i-w}, \ldots, e_i, \ldots, e_{i+w} \rangle. \qquad (26)$$

Then for some theoretical time $t$ and experimental time $e$ the position of $e$ within distribution $D_i$ is obtained wherein $D_i$ is selected such that its corresponding $t_i$ is closest to $t$. A percentile $q$ is obtained

$$q = \frac{|\{f \in D_i \mid f \leq e\}|}{|D_i|} \qquad (27)$$

representing where $e$ is within distribution $D_i$. It is of interest to remove an expected proportion of true results based on their retention time. It is assumed that a symmetric removal of those which are on both sides of the distribution is needed and then $q$ is converted to

$$p = 2 \cdot \left( \frac{1}{2} - \left| \frac{1}{2} - q \right| \right), \qquad (28)$$

which gives the required proportion. Thus, for instance, selecting results with $p \leq 0.1$ is then expected to give 10% of results with the largest differences (on both sides).

In tandem mass spectrometry, precursor spectra are often also measured and thus difference between the theoretical isotopic distribution and the observed one can be readily calculated as well. The difference can be also associated to statistical interpretation, analogously as for precursor mass difference. The software Isotopic Pattern Calculator (http://isotopatcalc.sourceforge.net/) can be used for the prediction of theoretical isotopic distributions.

Although multiple ways to calculate difference between distributions exist, we will utilize very simple one. For calculation of difference, peaks of theoretical and experimental distributions are initially mass-matched to some precursor mass tolerance (for instance 5 ppm on Orbitrap). Intensities of both distributions (experimental and theoretical) are normed to the sum to one and from this alignment the sum of squares is calculated from difference of intensities. The proportion of true results having at least as extreme difference as expected is then calculated. The calculation can be carried out in the same way as for precursor mass difference.

In the specific example of bottom-up proteomics, proteins are enzymatically digested into peptides and therefore in the resulting mixture it is expected that all peptides (of a particular protein) are present. This is called "protein evidence". It is therefore unlikely that just one peptide of a protein is identified, and this behavior may be modelled. Although multiple options for modelling of protein evidence exist, the modelling is restrained just to the presence or absence of different protein evidence (e.g., by assigning zero and one, respectively). Thus the probability of true match having $p$ as the extreme protein evidence is:

$$p = \frac{|\{r \in R \,|\, e_r \leq e\}|}{|R|}.$$

(29)

In practice, the $p \leq 0.1$ for no protein evidence and $p = 1$ for protein evidence. The task can be performed even before the step of protein inference, stating whether there exists a particular reference protein isoform for which there exists another peptide.

Additional/supporting criteria (e.g. precursor mass difference, retention time, isotopic distribution difference, protein evidence) can be combined to obtain a single criterium with a desirable statistical interpretation. This criterium is built in a way that it is expected to remove a desired proportion of true matches.

For the particular criteria $c_i$ of interest (e.g. precursor mass difference and retention time) of some peptide-spectrum match

$$\langle c_1, \ldots, c_n \rangle$$

(30)

a single value is calculated

$$c = \prod c_i.$$

(31)

For each $c$ the proportion $f(c)$ of the retained true matches among the results is calculated:

$$f(c) = \frac{|\{r \in R \,|\, c_r \leq c\}|}{|R|}$$

(32)

For a new instance of peptide-spectrum match with criteria

$$\langle d_1, \ldots d_n \rangle,$$

(33)

its single value $d$ is calculated as in (31), and then its value of $f(d)$. This can be done by looking up the closest value $c$ to $d$, and obtaining $f(c)$ (this, for example, equals the behavior of k-nearest neighbor with one neighbor). Of course, in this step various options exist, however, as often a lot of data is available, k-nearest neighbor with one neighbor is preferred due to simplicity and obvious interpretation. Then we can filter out the results with the expected loss of a desired proportion of true interpretations; based, however, on all additional/supporting criteria used.

The fourth step of the method of the invention relates to determining the identity of the analyzed entity.

Maximal probability $P_{max}$ of interpretations provides a rationale for removal of unlikely matches with a predictable long-term behaviour. For instance, selection of a large number $n$ of candidate interpretations with $P_{max} = p$ is expected to result with at most $n \cdot p$ correct interpretations. Therefore, the knowledge that a particular interpretation is less likely than a pre-determined value provides a rationale for its removal.

The most strict settings of $P_{max}$ corresponds to interpretations of $P_{max} = 1$, and are a preferred embodiment.

The probability P of candidate interpretations provides a rationale for selection of matches with a predictable long-term behaviour. For instance, selection of a large number $n$ of candidate interpretations with a probability higher than $p$, is expected to result in at least $n \cdot p$ correct interpretations.

The probabilistic interpretation for the additional/supporting criteria is built in a way to express how likely it is that the true interpretation has the supporting criteria as extreme as observed. If it is therefore unlikely (e.g., up to 10%) that the true interpretation would have as extreme criteria, then by removal of these interpretations it is expected that the same proportion (e.g., up to 10%) of correct matches can be removed.

In the following paragraphs, several uses of the method of the invention are described.

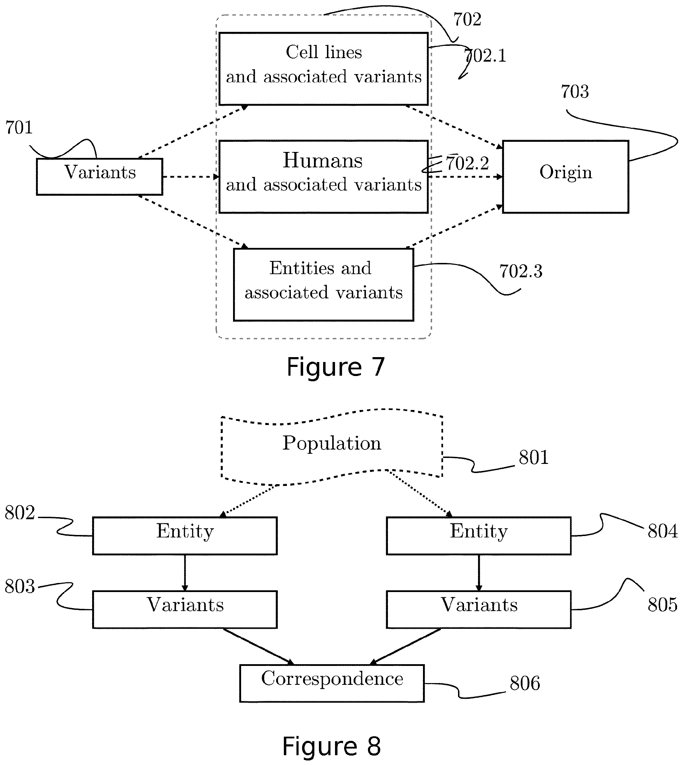

In one preferred embodiment, the method of the present invention can be utilized for matching to databases of origins. The following section describes matching of identified peptide or nucleic acid variants of known prevalence to database of origins, with each origin containing set of variants; Fig. 7 schematically describes the process.

For an analyzed sample $s$, we are interested in its true origin $\Gamma(s)$ and agreement $\Phi(s,C_i)$ of the sample $s$ and a candidate origin $C_i$ can be used for its establishment. Further, the sample s is considered as a set of variants $\{v_1,...,v_k\}$ identified in the sample s, denoted as

$$v(s) = \{v_1, \ldots, v_k\}. \tag{34}$$

The agreement $\Phi(s,C_i)$ can be, for instance, a number of matching variants. However, it is more preferable to define the agreement as

$$\Phi(\mathbb{C}_i, s) = \prod_{v \in \mathbb{C}_i \cap v(s)} \phi(v)^{-1}, \tag{35}$$

wherein $\varphi$ represents the prevalence of a variant, because it provides the probabilistic interpretation over the obtained results. Rescaling of (35) such that the sum over all $C_i$ equals one (the true origin is within the considered origins), gives the probability of the origin $C_i$ being the true origin:

$$P\Big(\Gamma(s) = \mathbb{C} \,\big|\, \Phi(\mathbb{C}, s) = x\Big). \tag{36}$$

Herein, a rationale for the use of the formula (35) for derivation of (36) is provided. Foremost, consider the agreement $\Phi(s,C_i)$ to be the actual matching variants:

$$\Phi(\mathbb{C}_i, s) = \mathbb{C}_i \cap v(s). \tag{37}$$

Said agreement (37) is used to enable derivation of the probability of determination of origin. Suppose, $v(s) = \{v_a,v_b\}$ variants identified in sample $s$ and suppose they are identified correctly (the true origin has these variants). Consider two origins

$$\mathbb{C}_a = \mathbb{C} \cup \{v_a\}, \mathbb{C}_b = \mathbb{C} \cup \{v_b\}, v_a \notin \mathbb{C}, v_b \notin \mathbb{C}, v_a \neq v_b. \tag{38}$$

Let us first set up the following notation: denote C+ all origins in a population having variants C. Probability of the true origin being within the sets of origins $\mathbb{C}_a^+$ and C+_b might be considered equal, given the same number of matching variants

$$P\Big(\Gamma(s) \in \mathbb{C}_a^+ \,\big|\, \Phi(\mathbb{C}_a, s) = \{v_a\}\Big) = P\Big(\Gamma(s) \in \mathbb{C}_b^+ \,\big|\, \Phi(\mathbb{C}_b, s) = \{v_b\}\Big) \tag{39}$$

Thus if the true origin is within C+_a or C+_b with the same probability, the size of both sets can be investigated. Suppose that the individual origins (within C+_a and C+_b) are equally likely *a priori*. Then the relative differences in the probability of the origins $C_a$, $C_b$ are inverse to number of entities having the corresponding variants:

$$\frac{P\left(\Gamma(s) = C_a\right)}{P\left(\Gamma(s) = C_b\right)} = \left(\frac{|C_a^+|}{|C_b^+|}\right)^{-1} \tag{40}$$

Furthermore, the relative difference between the number of the entities might be derived using population frequencies as:

$$\left(\frac{|C_a^+|}{|C_b^+|}\right)^{-1} = \frac{\prod_{v \in C_a} \phi(v)}{\prod_{v \in C_b} \phi(v)}. \tag{41}$$

The relative differences between the individual origins are preserved, and if the true origin is within the considered origins, they can be normalized to the value of one and thus (36) is readily established. Although, the assumption (39) was based on same number of matching variants, it might be assumed to hold, in general, for any sets of matching variants.

Another use of the method of the invention is in diagnosis of cancer by identifying somatic mutant peptides attributable to tumour in a sample taken from the body of a patient, e.g., blood or other fluids. The identification of the somatic mutant peptides attributable uniquely to tumour can be used for non-invasive diagnosis and monitoring of progression and recurrence of the disease.

For determination of the status of a variant (somatic or germline), various criteria can be used. Herein, global nucleotide alterations for the purpose are used.

Germline variants are considered as follows: a variant is present in dbSNP (v. 147), or ExAC (version of ExAC compilation without TCGA) and is preferably of a population frequency higher than $1.10^{-4}$ (in any of dbSNP or ExAC). Somatic variants are defined as those present in COSMIC, ICGC or TCGA, but not present in dbSNP and also not present in ExAC.

The presence of the somatic mutant protein variants (e.g., in blood of individual) can be in itself a sign of presence of a tumour. This is especially true for tumours with a high mutation rate, e.g., a melanoma.

For a more precise identification of the somatic mutant proteins exclusively attributable to tumour, the sample (e.g. of blood or blood plasma or blood serum or tears or urine or saliva or stool or breath condensate or lavages or effusions or liquors, etc.) of the patients may be analyzed before and after treatment (e.g., surgery, radiation, chemotherapy, biological therapy, immunotherapy, etc.). The drop in somatic mutant proteins after treatment establishes their exclusive correspondence to tumour and ultimately the tumour response. This can be done for establishing standards for such measurements or for the monitoring of a patient.

Another possible use of the method of the invention is in the monitoring the response of a recipient after transplantation by selection and quantification of peptides of the donor in a sample taken from the body of the recipient. Identification of increasing quantities of donor peptides in the sample (e.g. of blood or blood plasma or blood serum or tears or urine or saliva or stool or breath condensate or lavages or effusions or liquors, etc.) of the recipient is a sign of rejection or a risk of rejection of the transplanted organ.

Analysis of polymorphisms in the proteins is performed both on donor and recipient separately. Once these polymorphisms are associated with donors and recipients, identification of polymorphic peptides is uniquely linked to donor and recipient as well. Non-exclusive polymorphisms are not considered. The quantification can be done using any label-free quantification method, for example by integration of area under curve in LC/MS spectra. For precise quantification, targeted quantitative methods such as SRM/MRM can be used. Once polymorphisms are established and transitions of polymorphic peptides available, their monitoring in individuals can be readily performed.

Furthermore, calculation of pair-wise variant-based agreement between individuals is performed. The method is schematically shown in Fig. 8; the correspondence 806 between entities 802 and 804 based on their variants 803 and 805 is determined. Two samples $s_a, s_b$ from the tested individuals are analyzed and their agreement $\Phi(s_a, s_b)$ is determined using the method of the invention, optionally with a probabilistic interpretation. In the following description of the method, $\varphi$ denotes function from variant to its population frequency (such function might be for instance derived from population frequencies in dbSNP database).

The agreement may be based plainly on number of matching variants identified using particular methods $m_a, m_b$, for example, as follows:

$$\Phi(s_a, s_b) = |m_a(s_a) \cap m_b(s_b)| \tag{42}$$

Alternatively, the agreement may be in probabilistic terms. For the purpose, $\Gamma$ is a function from sample to its true origin, wherein the origin $e$ is a subset of all variants (neglecting the possibility that two distinct origins have the same variants). The probability of two samples having the same origin, given the observed agreement, is:

$$\mathrm{P}\Big(\Gamma_a(s_a) = \Gamma_b(s_b) \mid \Phi(s_a, s_b) = x\Big). \qquad (43)$$

Yet alternatively, the probability of at least as extreme match as $x$ at random may be used:

$$\mathrm{P}\Big(\Phi(s_a, s_b) \geq x\Big). \qquad (44)$$

Method $m$ of identification of variants applied to a sample may identify exactly the variants in origin, and the origin is the same if and only if the identified variants are equal in both samples, however such situation is less likely in practice.

In some embodiments, the method $m$ applied to the sample identifies a proportion $r$ of variants in the sample. The proportion might be unknown in advance (or it might depend on the concentration of the sample, etc.), but the fact that samples are drawn from a known population may be utilized for its derivation. In such case, the expected number of variants in the sample with known population frequencies is

$$\sum \phi(v) = n. \qquad (45)$$

The probability of both the presence of variant v and its identification using method $m$ is designated as $\mathrm{P}^{m+}(v)$.

In some embodiments, identification of a variant might be independent of the variant itself, and therefore the probability of the identification is equal for each variant. In other embodiments, the probabilities may be different. Nevertheless, if $n$ variants were identified using the method $m$ in a sample, then the probabilities of identification can be expressed as the expected number of identified variants being the actual number of identified variants:

$$\sum \mathrm{P}^{m+}(v) = n. \qquad (46)$$

In shotgun proteomics, it is beneficial to model the probability of identification of a variant. Such identification might be modelled as a function of variant abundance because more abundant proteins are more likely to be measured. This is also important for establishment of agreement due to chance, because identification of variants of high population frequency in highly abundant proteins is quite likely even for random individuals. The probability of identification of a variant can be modelled on per-protein basis as its abundance. However, it is preferred to model it as a coverage $C(p)$ of protein $p$ (by identified reference peptides), which effectively normalizes the abundance within zero-one range. Proteins which are highly covered by peptides give a high probability of identification of a variant (if the variant is present) as opposed to proteins with a low coverage. For further simplification, the coverage might be calculated over genes and restricted to peptides uniquely alignable to genes (around 90%). The coverage for a gene might be then defined as the average of coverages of proteins (corresponding to the gene). This is followed by a further normalization of the probabilities of identification, such that (46) holds, as follows:

$$\mathrm{P}^{m+}(v) = \frac{C(v) \cdot \phi(v)}{\sum\limits_{v} \big(C(v) \cdot \phi(v)\big)} \cdot n, \qquad (47)$$

Depending on the agreement model, at least as good match (44) may be calculated using different approaches. In general, the probability may be numerically calculated using viable methods, for example, Monte Carlo simulation. The following paragraphs focus on number of matching variants (42).

In general, the probability of a match of particular variant v at random using methods $m_a, m_b$ is

$$\mathrm{P}^{m_a}(v) \cdot \mathrm{P}^{m_b}(v), \qquad (48)$$

if these events are statistically independent. The situation is, however, more complicated in shotgun proteomics, because some peptides are more suitable for identification (e.g. due to ionisation characteristics) than others. In other words, identification of variant in first sample changes, usually increases, the probability of identification of a variant in the second sample.

This effect can be modelled on pairwise comparison of coverage of matching proteins in both samples. Suppose $C_a(p)$ is a coverage of protein $p$ in sample $a$ and $C_b(p)$ in sample $b$. The expected shared coverage if distributed uniformly is $C_a(p) \cdot C_b(p)$. However, as there are preferences for individual peptides, the actual shared coverage is usually higher. The relationship (example of which is visualized in Fig. 42), could be modelled in variety of ways. Given the large set of available data, it can be modelled also using k-nearest neighbor regression. Here, the regression model is represented as a function $k$ (5 neighbors, Euclidean distance). Therefore instead of multiplication of probabilities as in (48), it is calculated as

$$k\left(\mathrm{P}^{m_a+}(v), \mathrm{P}^{m_b+}(v)\right). \qquad (49)$$

If the agreement model is a number of matching variants (42), the calculation of (44) may be approximated, for example, using Binomial distribution, with the probability of success in one trial being equal to mean value of (49) over all variants.

The determination of the identity of the entity solves the problems of interpretation of mass spectra commonly encountered in shotgun proteomics and many other fields.

The method of the present invention may be also used for determination of identity, in particular for authentication of cell lines or identification of an individual from mass spectra of proteome.

The method may be also used for identification of non-host organism from mass spectra of proteome of host organism, in particular for diagnosis of microbial infection or colonization.

The method may be also used for identification of presence of a tumour from mass spectra of body fluid proteins or estimation of tumour characteristics through presence or absence of somatic mutations.

The method may be also used for monitoring of organ transplantation and early detection of transplant rejection from mass spectra of biological materials of recipient.

## Examples of carrying out the invention

### Example 1 - Determination of identity of an entity

*Collecting analytical data*

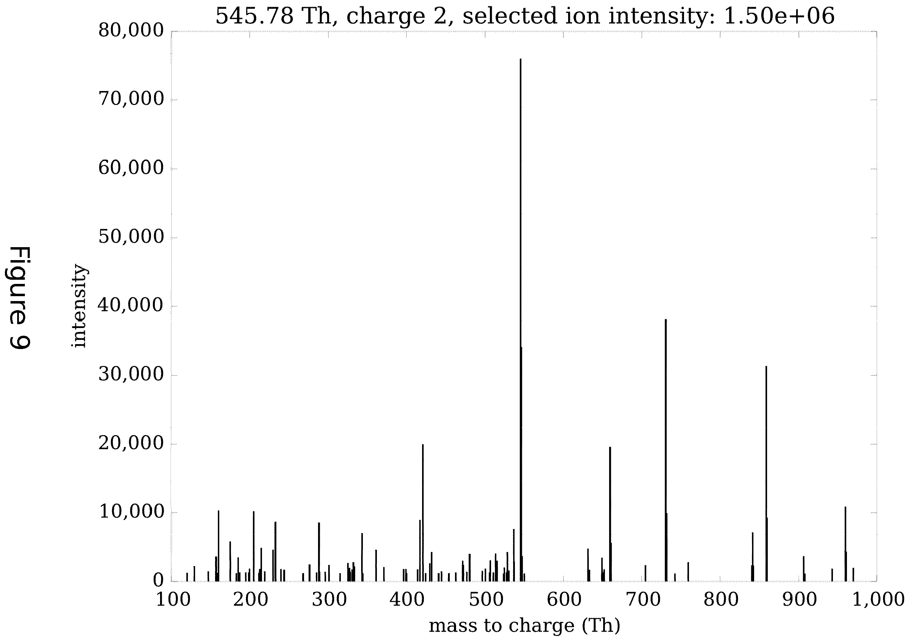

This example illustrates fragment mass spectrum of analytical data collected for unknown peptide in shotgun proteomics. This particular example of MS/MS spectrum is shown on Fig. 9 and the process of determination of entity is further illustrated on it.

*Obtaining candidate identity by enumeration*

The candidate identities for spectrum on Fig. 9 are obtained through enumeration, whose description follows. The probabilities for cleavage after particular amino acid (even modified) are specified in Fig. 6b. Probabilities of few modifications were set as in Fig. 6c. The rest of modifications (but not substitutions), were set prior-like probability of 0.001. The prior-like probability of amino acid substitution was set to be dependent on the least number of nucleotide substitutions of given codon. If the substitution can happen (for any combination of codons) in one nucleotide alteration, it is $0.0002 = q$, otherwise its powers; thus if $n$ is the minimum number of nucleotide substitutions, then the prior-like probability is $n^q$. The prior-like probabilities of coded amino acids and terminals were set such that sum over their prior-like probability of amino acid and all its modifications equals one. Small partial list of modifications of amino-acids, along with their prior-like probabilities is illustrated in the following table:

| modification | amino acids | prior-like |
|---|---|---|
|  | [C] | 0.0000 |

(continued)

| modification | amino acids | prior-like |
|---|---|---|
| | [M] | 0.6828 |
| | [K] | 0.8186 |
| | [N-term] | 0.9080 |
| | [S] | 0.9156 |
| | [T] | 0.9314 |
| | [Y] | 0.9338 |
| | [N] | 0.9496 |
| | [R] | 0.9566 |
| | [H] | 0.9626 |
| | [C-term] | 0.9670 |
| | [D] | 0.9686 |
| | [E] | 0.9688 |
| | [W] | 0.9800 |
| | [P] | 0.9826 |
| | [F] | 0.9848 |
| | [Q] | 0.9858 |
| | [L] | 0.9900 |
| | [G] | 0.9914 |
| | [I] | 0.9942 |
| | [V] | 0.9954 |
| | [A] | 0.9966 |
| Methyl | [N-term, E, D, C-term, L, I, R, Q, N, K, H, C] | 0.0010 |
| Methyl | [T, S] | 0.0100 |
| Oxidation | [W, H, C, R, Y, F, P, N, K, D, G] | 0.0010 |
| Oxidation | [M] | 0.3000 |
| Phospho | [R, C, D, Y, H, T, S] | 0.0010 |
| ... | ... | ... |

In the next step, all candidate identities of prevalence higher than $4 \cdot 10^{-6}$ were obtained, and only candidates within 5 ppm (parts-per-million) of calculated precursor mass were considered and are ordered from highest prevalence, an excerpt shown in the following table:

| sequence | prevalence | ppm |
|---|---|---|
| R.-FYPHDVLSL-.P (SEQ ID NO.1) | $1.1 \cdot 10^{-3}$ | 2.859482 |
| S.-MVELWAWR-.E (SEQ ID NO.2) | $8.8 \cdot 10^{-4}$ | -3.124660 |
| R.-SPS(Methyl)GFGDPGKK-.D (SEQ ID NO.3) | $8.5 \cdot 10^{-4}$ | -0.839305 |
| R.-MTQALALQAGS-.L (SEQ ID NO.4) | $8.0 \cdot 10^{-4}$ | 2.272081 |

(continued)

| sequence | prevalence | ppm |
|---|---|---|
| D.-NTVVMEEIR-.R (SEQ ID NO.5) | 7.8 · 10-4 | 2.272081 |
| T.-AISNASDVWK-.K (SEQ ID NO.6) | 7.3 · 10-4 | -0.830127 |
| R.-(Formyl)ANAFLEELR-.P (SEQ ID NO.7) | 5.6 · 10-4 | -0.834716 |

The precursor mass difference of 5 ppm was selected due to accuracy of the employed mass spectrometer (Orbitrap Elite). Depending on experimental conditions, the precursor tolerance can be much wider (e.g., 500 Da) as is the case of open search or total (all candidate identities independent of precursor mass are considered). In these cases, the mass difference is further localized (or decomposed into multiple modifications and their localization) as is usual in open search, but prevalences of candidate identities with localized masses are further updated by corresponding prevalences of modifications.

The limiting prevalence of $4.10^{-6}$ should be low enough for most experiments at present. The number of spectra per experiment is in order of hundreds of thousands; in such case we would expect at most few peptides identified if lower minimal prevalence was taken into account.

*Calculating score*

*Agreement*

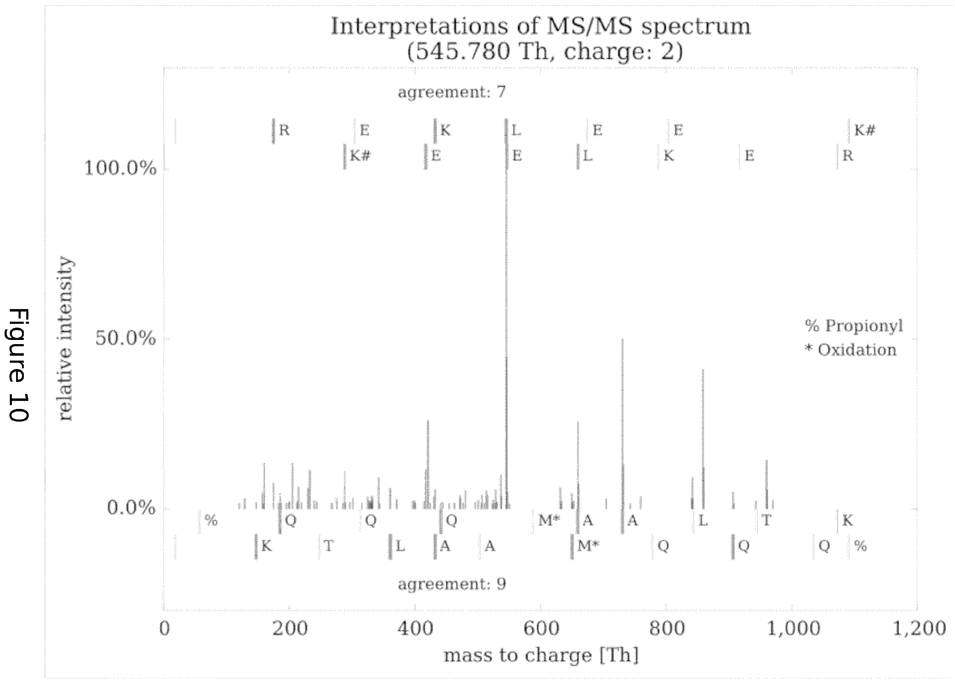

This section describes agreement of theoretical spectrum of peptide and experimental (measured) spectrum. Number of matching peaks (of experimental and theoretical spectrum) is used as particular agreement model (Fig. 10). In this example, only singly charged ions (b, y) are used for prediction of theoretical spectrum. The agreement in Fig. 10 is shown for two peptides (from those enumerated in previous step), placed at top and bottom separately. Prefix (b) ions are shown closer to the MS/MS spectrum and suffix (y) ions are shown further (both on top and bottom). Ions matching experimental spectrum (fragment tolerance of 0.3 Da), are thicker. The agreement corresponds to total number of matching peaks. The agreement of individual peptides is illustrated in the following table, in which the first few peptides are ordered from highest spectral match.

| sequence | agreement | prevalence | ppm |
|---|---|---|---|
| K.-M(A→T) TQALEELR-.S (SEQ ID NO.8) | 12 | 5.7 · 10-5 | 2.272081 |
| R.-(Carboxymethyl) TSGGAGGLGSLR-.A (SEQ ID NO.9) | 11 | 4.8 · 10-4 | -4.529832 |
| K.-(Methyl)M(Oxidation)AQALEELR-.S (SEQ ID NO.10) | 11 | 1.5 · 10-4 | 2.267492 |
| R.-SL(R→M) MVDIAEGR-.K (SEQ ID NO.11) | 11 | 6.8 · 10-5 | 2.262903 |
| K.-LV(K→M)MDSIAEGR-.I (SEQ ID NO.12) | 11 | 5.6 · 10-5 | 2.262903 |
| R.-TMQALEIE(L→R) R-.Q (SEQ ID NO.13) | 10 | 6.9 · 10-5 | 2.272081 |
| .-MAS(Acetyl) S(Methyl) LLAGER-.L (SEQ ID NO.14) | 10 | 4.5 · 10-6 | 2.267492 |

*Maximal probability*

The following table illustrates determination of identity using maximal probability of candidate identities ($P_{max}$ column), calculated from agreement and prior-like probabilities.

| sequence | agreement | prevalence | Pmax |
|---|---|---|---|
| K.-M(A→T) TQALEELR-.S (SEQ ID NO.8) | 12 | 5.7 · 10-5 | 1 |
| R.-(Carboxymethyl)TSGGAGGLGSLR-.A (SEQ ID NO.9) | 11 | 4.8 · 10-4 | 1 |
| K.-(Methyl) M(Oxidation) AQALEELR-.S (SEQ ID NO.10) | 11 | 1.5 · 10-4 | 0.235 |
| R.-SL(R→M) MVDIAEGR-.K (SEQ ID NO.11) | 11 | 6.8 · 10-5 | 0.098 |

(continued)

| sequence | agreement | prevalence | Pmax |
|---|---|---|---|
| K.-LV(K→M) MDSIAEGR-.I (SEQ ID NO.12) | 11 | 5.6 · 10-5 | 0.069 |
| K.-DC(GlyGly)VLNATLK-.Q (SEQ ID NO.15) | 10 | 2.9 · 10-4 | 0.377 |

*Probability*

The following description explains the association of probability of correctness (P) using Bayes' Theorem. For this purpose, model of true agreement and random agreement is defined.

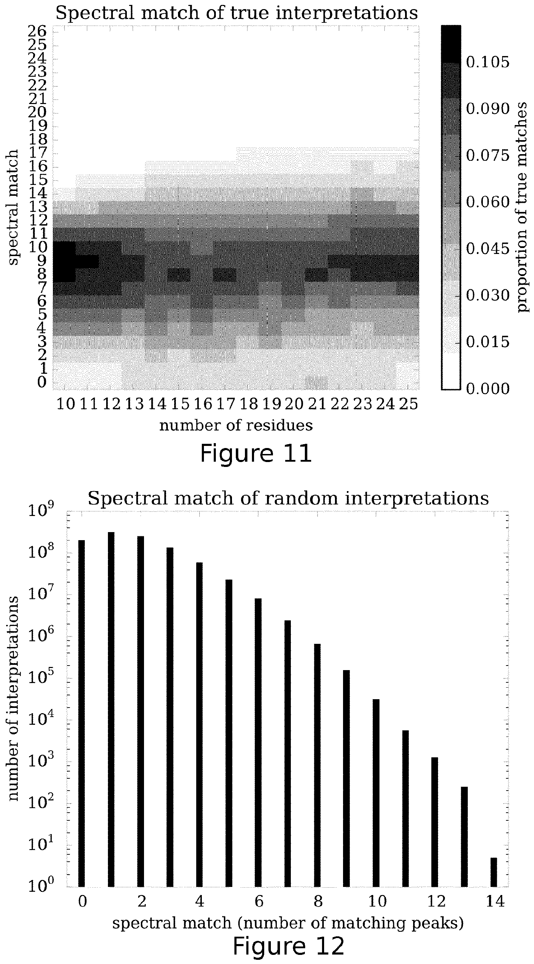

Agreement of true interpretations is modelled as follows. Agreement is evaluated on interpreted spectra from spectral database of X!Hunter, which are assumed to be true interpretations. The behaviour (Fig. 11) is shown only for doubly charged fragment mass spectra. In this example, the model is taken as an average behaviour over number of residues. This is meaningful, as the behaviour over number of residues is quite independent on the length of peptide.

Agreement of random interpretations for this spectrum is visualized on Fig. 12. In this example, random agreement is modelled in such way that the probability of random match decreases $20\times$ with increasing number of matching peaks.

In derivation of prior probability from prior-like probabilities, the c in (23) equals 1.0 (this is assumption that true interpretation is within the candidates), which enables establishment of probability. The associated probability of candidate identities is shown in the following table:

| sequence | agreement | prevalence | $P_{true}$ | $P_{random}$ | $P_{prior}$ | P |
|---|---|---|---|---|---|---|
| K.-M(A→T)TQALEELR-.S (SEQ ID NO.8) | 12 | 5. 7 · 10-5 | 0.066353 | 8.871817e-09 | 0.002357 | 0.520089 |
| R.-(Carboxymethyl) TSGGAGGLGSLR-.A (SEQ ID NO.9) | 11 | 4.8 · 10-4 | 0.082515 | 1.774363e-07 | 0.019913 | 0.273163 |
| K.-(Methyl) M(Oxidation) AQALEELR-.S (SEQ ID NO.10) | 11 | 1.5 · 10-4 | 0.082515 | 1.774363e-07 | 0.006103 | 0.083718 |
| R.-SL(R→M) MVDIAEGR-.K (SEQ ID NO.11) | 11 | 6.8 · 10-5 | 0.082515 | 1.77 4363e-07 | 0.002823 | 0.038721 |
| K.-LV(K→M) MDSIAEGR-.I (SEQ ID NO.12) | 11 | 5.6 · 10-5 | 0.082515 | 1.77 4363e-07 | 0.002325 | 0.031888 |
| K.-DC(GlyGly) VLNATLK-.Q (SEQ ID NO. 15) | 10 | 2.9 · 10-4 | 0.103277 | 3.548727e-06 | 0.012038 | 0.010334 |
| K.-DCVLNATLK-.Q (SEQ ID NO.16) | 10 | 2.7 · 10-4 | 0.103277 | 3.548727e-06 | 0.011107 | 0.009535 |

*Additional/supporting criteria*

The run corresponding to the analytical data was used for selection of true interpretation as explained earlier (tryptic reference human peptides, 5 ppm precursor mass difference, statistical significance of 0.1).

*Precursor mass difference*

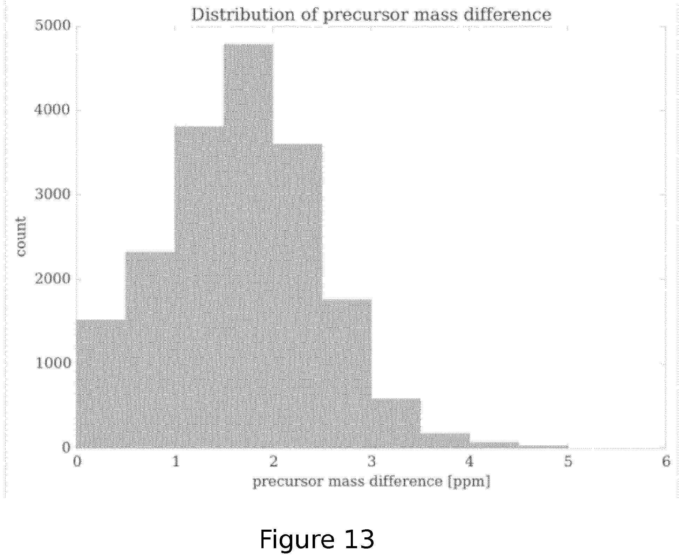

The Fig. 13 shows the distribution of precursor mass differences for true interpretations.

*Retention time*

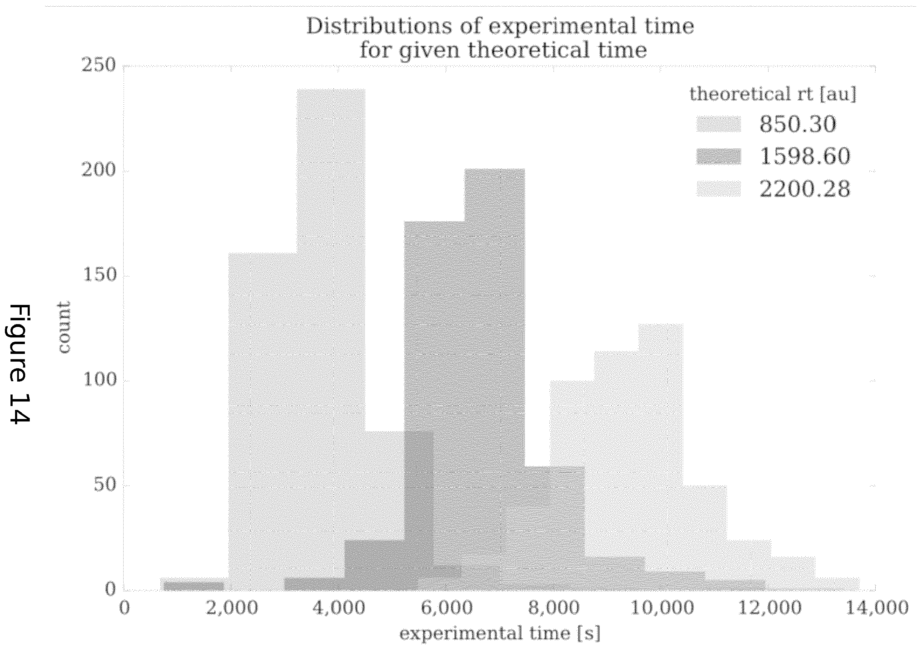

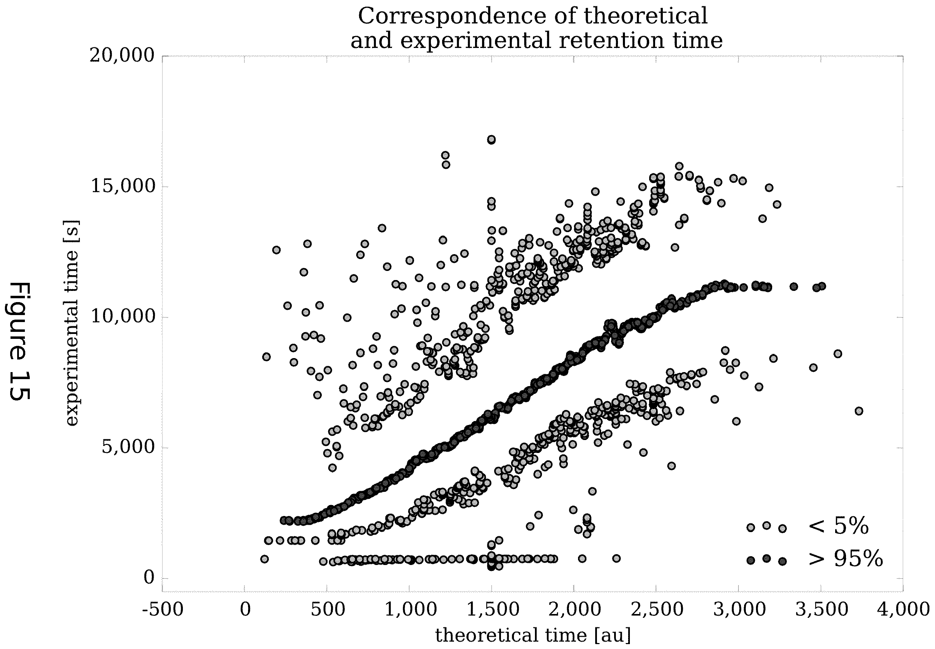

The Fig. 14 shows the distribution of experimental time for particular predicted theoretical time and its neighbors (the theoretical time is significantly shifted). With the assumption of symmetric difference, the Fig. 15 shows selection of interpretations near tails of the distribution (<5%) and those in the center (>95%).

*Isotopic distribution difference*

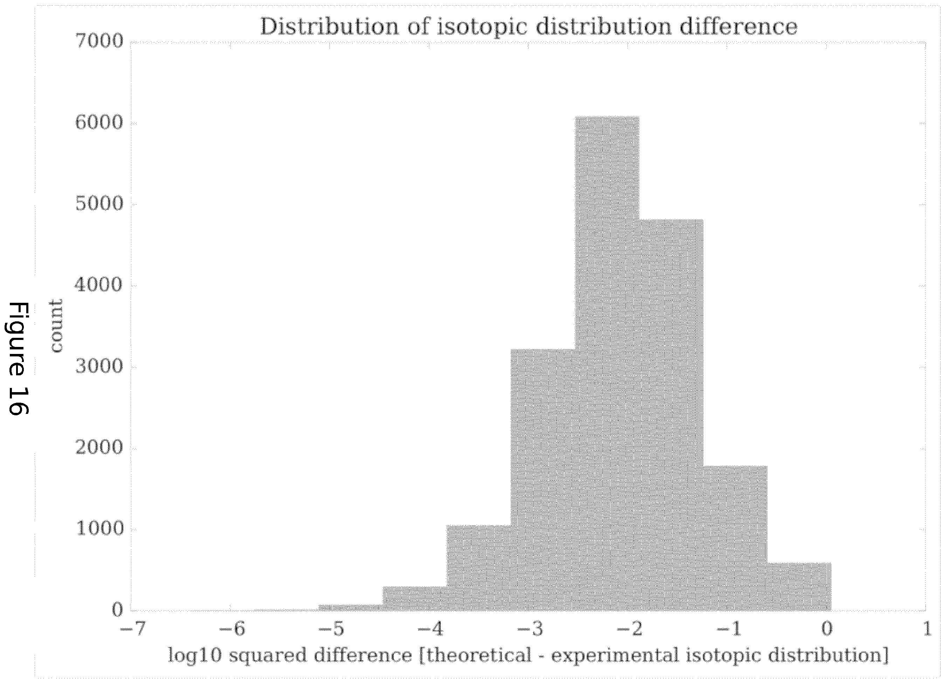

Similarly as for precursor mass difference, Fig. 16 shows the distribution of differences between theoretical and experimental isotopic distribution.

*Protein evidence*

In case of protein evidence, 8.129% of assumed true interpretations were without existence of other peptide from the same protein.

*Combination of additional/supporting criteria*

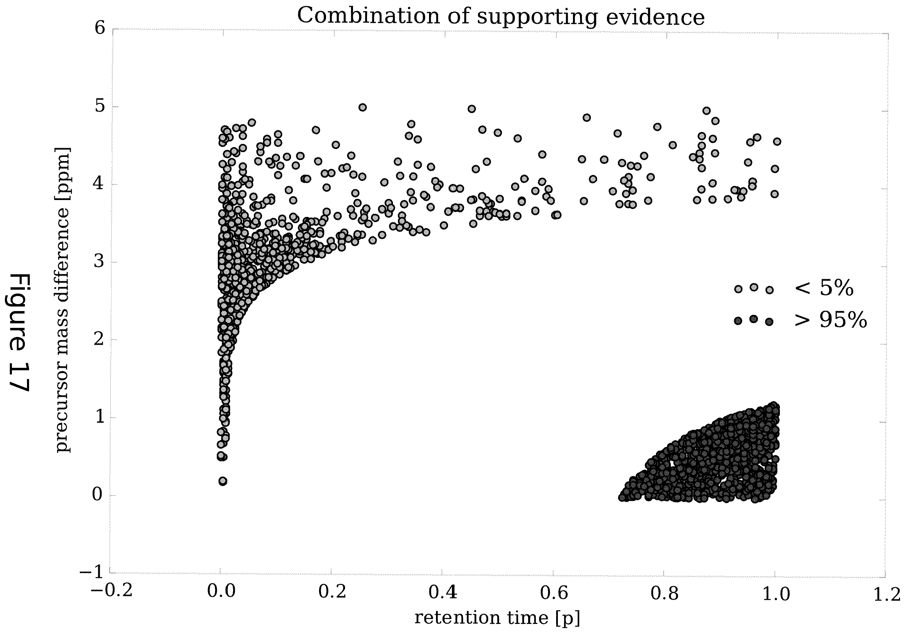

The Fig. 17 shows combination of supporting evidence, split for unlikely ($\leq$ 5%) and likely ($\geq$ 95%) results. For instance, in case of likely results, it can be seen that as retention time is getting closer to the center of the distribution (p closer to one), the precursor mass difference can be higher to still obtain probability greater than 95%. Therefore the figure captures the numerical relationship between these supporting criteria and the resulting probability.

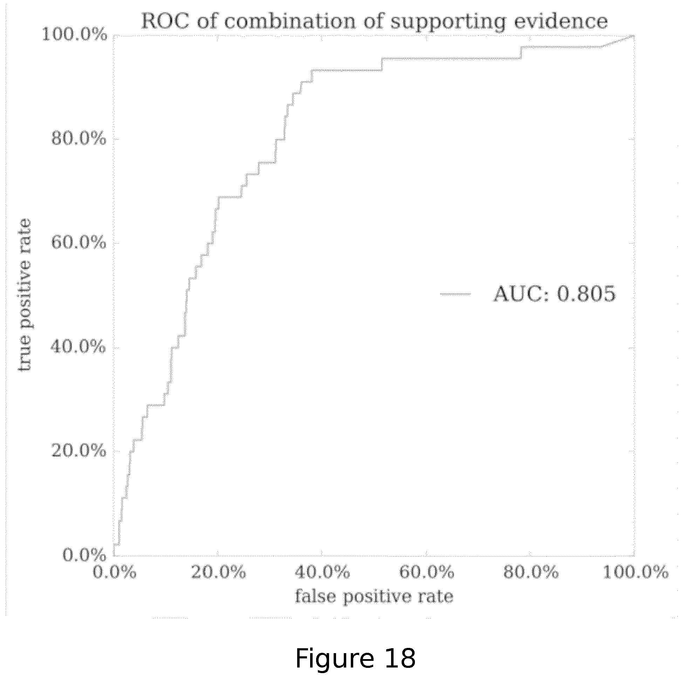

The ROC curve on Fig. 18 shows capabilities of removal of incorrect interpretations with the use of supporting evidence. The filtering is evaluated on interpretations of variant peptides (statistical spectral significance E-Value of 0.1 in X!Tandem). In the ROC curve, true interpretations are assumed to be those which have sequencing support (variant also found in sequencing). It is clear that supporting evidence can help in removal of incorrect interpretations. For instance, here around 50% of sequencing unsupported results is removed, while retaining around 90% of sequencing supported results.

The following table contains associated evidence from supporting criteria and their combined values.

| sequence | prevalence | agreement | Pmax | precursor mass p | retention p | protein p | isotopic p | comb. p |
|---|---|---|---|---|---|---|---|---|
| K.-M(A→T)TQALEELR-.S (SEQ ID NO.8) | 5.7 · 10-5 | 12 | 1 | 0.219550 | 0.712 | 1.000000 | 0.389195 | 0.479031 |
| K.-(Methyl)M(Oxidation)AQALEELR-.S (SEQ ID NO.10) | 1.5 · 10-4 | 11 | 0.235 | 0.221310 | 0.552 | 1.000000 | 0.389195 | 0.427736 |
| R.-SL(R→M) MVDIAEGR-.K (SEQ ID NO.11) | 6.8 · 10-5 | 11 | 0.098 | 0.222536 | 0.116 | 1.000000 | 0.389195 | 0.196714 |
| K.-LV(K→M)MDSIAEGR-.I (SEQ ID NO.12) | 5.6 · 10-5 | 11 | 0.069 | 0.222536 | 0.436 | 0.081207 | 0.389195 | 0.099081 |
| R.-(Carboxymethyl)TSGGAGGLGSLR-.A (SEQ ID NO.9) | 4.8 · 10-4 | 11 | 1 | 0.001813 | 0.604 | 1.000000 | 0.970927 | 0.053857 |
| K.-M(Oxidation) AQ(Methyl)ALEELR-.S (SEQ ID NO.17) | 1.3 · 10-4 | 10 | 0.086 | 0.221310 | 0.552 | 1.000000 | 0.389195 | 0.427736 |
| R.-TS(G→D) DGAGGLGSLR-.A (SEQ ID NO.18) | 8.5 · 10-5 | 10 | 0.049 | 0.001813 | 0.636 | 1.000000 | 0.970927 | 0.055138 |

The combined *p* can be used for removal of matches which are unlikely to be correct. In this case, selecting expected removal of 10% of correct results, the first interpretation (highest scoring from viewpoint of spectral match) is not removed.

*Determining identity*

*Maximal probability*

Determination of identity in this example is based on selecting interpretation which is of maximal agreement and has $P_{max}= 1$. Such interpretation can be at most one, and it is the best candidate for given agreement and prevalence modeling; it is the first one in the previous table.

*Probability*

Determination of identity in this example is based on selection of the interpretation which has higher probability than 0.5; such interpretation can be at most one and it is the most likely interpretation. In this example, it is again the first one and the identity determined is the same as in previous example using $P_{max}$ and highest agreement.

**Example 2 - Identification of observed mutant and polymorphic proteins**

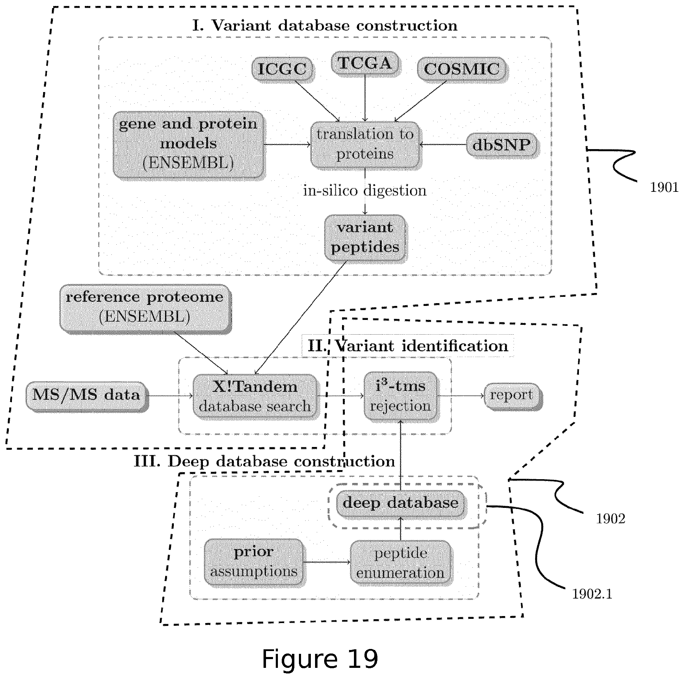

The system overview (Fig. 19) represents example 102 of incorporation of prevalence model (Fig. 1) in shotgun proteomics for identification of variant peptides. In this example, candidate identities are first scored in database search and further reevaluated with the use of prevalence to obtain maximal probability of their correctness.

In general, the identification system 1901 corresponds to 102.1 and the rejection system 1902 corresponds to system comprising prevalence model 102.2. The search database for X!Tandem is represented in form of variant peptide fasta file constructed by translation of variant mRNA, and excerpt from it looks as follows:

```
>ID-00000000 none
NEIPIR (SEQ ID NO.19)
>ID-00000001 none
AAVAAITQALVGR (SEQ ID NO.20)
>ID-00000002 none
SPPLPGDLGGPSK (SEQ ID NO.21)
>ID-00000003 none
LSAAQTNGGGSAGMEGIMNPYTALPTPQQLLAIEQSVYSSDPFR (SEQ ID NO.22)
>ID-00000004 none
NTEILTGSWSDQTYPEGTHAIYK (SEQ ID NO.23)
```

The deep database 1902.1 corresponds to prevalence model, was obtained through enumeration (Fig. 6) and was stored as a peptide database, along with prior-like probabilities. It is preferred to store the database and index it by precursor mass, because the interpretations will be loaded for given precursor mass range; an excerpt of such record is shown here:

| precursor mass | position | prior-like | protein | sequence |
|---|---|---|---|---|
| 1322.659981 | 540 | 0.000004 | ENSP00000364543 | QPT(Methyl)HQGGPAQDR(Methyl:2H(3) 13C(1)) (SEQ ID NO.24) |
| 1322.659984 | 539 | 0.000058 | ENSP00000292377 | GGGGSARY(iTRAQ4plex114)NQGR (SEQ ID NO.25) |
| 1322.659984 | 539 | 0.000059 | ENSP00000292377 | (iTRAQ4plex114)GGGGSARYNQGR (SEQ ID NO.26) |
| 1322.659984 | 512 | 0.000484 | ENSP00000400142 | (iTRAQ4plex114)GNPSGGGNVQHR (SEQ ID NO.27) |
| 1322.659991 | 179 | 0.000479 | ENSP00000211998 | QQELTHQEHR(Methyl:2H(3)13C(1)) (SEQ ID NO.28) |

Further, it is preferred to construct the database first for a wide range of masses (for example, 700-2500 Da) and further index peptides into smaller ranges (for example, 0.01 Da), to save computational time.

The rejection system 1902 is an example of incorporation of prevalence model for reevaluation of candidate identities (Fig. 2), corresponding to 203.B. Rejection designates the reevaluation of candidate identities, in which maximal probability of correctness of candidate identity is evaluated and used for rejection of candidates.

*Obtaining candidate identities, Database search in peptide database of variants*

The process is illustrated step by step on identification of variant peptides on samples measured on colorectal cancer cell line HCT116. The steps can be split into three phases: i) spectral match using database search, ii) assignment of additional information, iii) obtaining additional candidate identities. In the first step, the variant peptide database is searched using database search method, herein using X!Tandem. Matching of spectra and variant peptides gives initial results, an example is illustrated in the following table, ordered by most significant matches first (E-Value).

| MZ | sequence | [XTandem] scoreT | E-Value |
|---|---|---|---|
| 945.122864 | GSGDPSSSSSSGNPIVYLDVDANGKPLGR (SEQ ID NO. 29) | 107.099998 | 1.000000e-15 |
| 912.987976 | IGLIGGTGLDDPEILEGR (SEQ ID NO.30) | 77.800003 | 1.000000e-15 |
| 1156.580444 | VEGTEPTTAFNIFVGNLNFNK (SEQ ID NO.31) | 72.400002 | 2.700000e-15 |
| 978.494324 | GEELGGGQDPVQLISGFPR (SEQ ID NO.32) | 98.500000 | 3.900000e-15 |
| 593.311707 | HPLHVTYAGAAVDELGK (SEQ ID NO.33) | 77.900002 | 5.500000e-15 |
| 975.565063 | VPIASQGLGPGSTVLLVVDK (SEQ ID NO.34) | 80.599998 | 8.800000e-15 |
| 1045.49340 | (Gln-¿pyro-Glu)QSGGSSQAGAVTVSDVQELMR (SEQ ID NO.35) | 70.900002 | 7.900000e-14 |
| 893.930054 | DPGGLTAGSTDEPPMLTK (SEQ ID NO.36) | 75.400002 | 1.500000e-13 |
| 893.929443 | DPGGLTAGSTDEPPMLTK (SEQ ID NO.37) | 83.699997 | 6.000000e-13 |
| 1174.115967 | NFYGGNGIVGAQVPLGAGIALAC(Carbamidomethyl)K (SEQ ID NO.38) | 57.900002 | 1.100000e-12 |
| 889.464172 | HPLHVTYAGAAVDELGK (SEQ ID NO.39) | 77.500000 | 1.400000e-12 |
| 908.914551 | TVSGTC(Carbamidomethyl)GPGQPASSSGGPGR (SEQ ID NO.40) | 79.199997 | 1.600000e-12 |
| 1079.080811 | NSNPVIAELSQAINSGTLLSK (SEQ ID NO.41) | 77.599998 | 1.700000e-12 |
| 971.964539 | VWLDPNETNELANANSR (SEQ ID NO.42) | 73.500000 | 1.900000e-12 |

There are many highly significant matches of variant peptides, which however, does not mean they are correct interpretations.

In the second step, variant peptides are aligned to reference protein-coding sequences (ENSEMBL, human genome), their distance to reference genome is calculated and additional information attached. Only reference peptides which can be result of a single nucleotide variation are considered in this example (this is also because prevalence of such peptides is much higher and simplifies identification task). Furthermore, here, only peptides which can be aligned to one genomic location are considered (such decision has some benefits, for example, it is easier to establish peptide-derived nucleotide variation, which has further benefits of deriving population frequency, or calculating correspondence to nucleotide sequencing of matching sample). Excerpt of results of this procedure is illustrated by the following table:

| E-Value | sequence | substitution | candidate SNVs |
|---|---|---|---|
| 1.000000e-15 | GSGDPSSSSSSGNPIVYLDVDANGKPLGR (SEQ ID NO.43) | (L, I) | [(chrIO, 81107442, 81107442, C, A)] |

(continued)

| E-Value | sequence | substitution | candidate SNVs |
|---|---|---|---|
| 2.700000e-15 | VEGTEPTTAFNIFVGNLNFNK (SEQ ID NO.44) | (L, I) | [(chr2, 232324977, 232324977, C, A)] |
| 3.900000e-15 | GEELGGGQDPVQLISGFPR (SEQ ID NO.45) | (L, I) | [(chr11, 62444347, 62444347, C, A)] |
| 8.800000e-15 | VPIASQGLGPGSTVLLVVDK (SEQ ID NO.46) | (L, I) | [(chr1, 949537, 949537, C, A)] |
| 1.500000e-13 | DPGGLTAGSTDEPPMLTK (SEQ ID NO.47) | (I, L) | [(chr16, 30900254, 30900254, A, C), (chr16, 30900254, 30900254, A, T)] |
| 1.700000e-11 | VEHLEEGPMLEQLSK (SEQ ID NO.48) | (I, L) | [(chr12, 93881377, 93881377, A, C), (chr12, 93881377, 93881377, A, T)] |
| 1.800000e-11 | IVVECVMNNVTCTR (SEQ ID NO.49) | (L, I) | [(chr8, 82196200, 82196200, T, A)] |
| 9.100000e-11 | GVEAANVTGPGGVPVQGSK (SEQ ID NO.50) | (A, V) | [(chr1, 43162316, 43162316, C, T)] |
| 1.000000e-10 | DMLLEIEEQLAESR (SEQ ID NO.51) | (L, I) | [(chr2, 238662071, 238662071, C, A)] |
| 1.100000e-10 | IVVECVMNNVTCTR (SEQ ID NO.52) | (L, I) | [(chr8, 82196200, 82196200, T, A)] |
| 1.200000e-10 | VNNSSLIGLGYTQTIKPGIK (SEQ ID NO.53) | (L, I) | [(chr5, 133309490, 133309490, C, A)] |
| 1.300000e-10 | SPASDTYIVFGEVK (SEQ ID NO.54) | (A, V) | [(chr12, 57107324, 57107324, C, T)] |
| 1.400000e-10 | IVVECVMNNVTCTR (SEQ ID NO.55) | (L, I) | [(chr8, 82196200, 82196200, T, A)] |
| 1.500000e-10 | IVVECVMNNVTCTR (SEQ ID NO.56) | (L, I) | [(chr8, 82196200, 82196200, T, A)] |
| 1.700000e-10 | IEAGEGETAVLNQLQEK (SEQ ID NO.57) | (Q, E) | [(chr12, 93213151, 93213151, C, G)] |
| 2.000000e-10 | DMDLTGCLESGGSEEPGGIGVGEK (SEQ ID NO.58) | (N, D) | [(chr11, 57076574, 57076574, A, G)] |
| 2.200000e-10 | LSYDQQQQQQQQQQQQQQAIQSR (SEQ ID NO.59) | (L, I) | [(chr17, 17697138, 17697138, C, A)] |

In the third step, for each spectrum with variant peptide interpretation, all candidate identities (minimal prior-like probability of $4 \cdot 10^{-6}$) are enumerated (as described previously).

*Calculating score - Maximal probability*

Maximal probability of interpretations is established. Results of this procedure are illustrated in the following table:

| sequence | E-Value | substitution | sequencing support | Pmax |
|---|---|---|---|---|
| GEELGGGQDPVQLISGFPR (SEQ ID NO.60) | 3.900000e-15 | (L, I) | | 0.000201 |
| VPIASQGLGPGSTVLLVVDK (SEQ ID NO.61) | 8.800000e-15 | (L, I) | | 0.000201 |
| DPGGLTAGSTDEPPMLTK (SEQ ID NO.62) | 1.500000e-13 | (I, L) | | 0.000199 |
| VWLDPNETNELANANSR (SEQ ID NO.63) | 1.900000e-12 | (I, L) | | 0.000199 |
| ALDVGSGSGLLTAC(Carbamidomethyl)FAR (SEQ ID NO.64) | 4.400000e-12 | (I, L) | | 0.000199 |
| NSGPIGTEM(Oxidation)NTGFSSEVK (SEQ ID NO.65) | 6.900000e-12 | (L, I) | | 0.000201 |
| FLQEHGSDSFIAEHK (SEQ ID NO.66) | 9.000000e-09 | (L, I) | | 0.000201 |
| TAMNVNEIFMALAK (SEQ ID NO.67) | 9.800000e-09 | (I, L) | | 0.000199 |
| SC(Carbamidomethyl)QTALVEILDVIAR (SEQ ID NO.68) | 1.100000e-08 | (V, A) | X | 1.000000 |
| WSGPLSLQEVDEQPK (SEQ ID NO.69) | 1.200000e-08 | (Q, K) | X | 1.000000 |
| NLTNPNTVIIIIGNK (SEQ ID NO.70) | 1.200000e-08 | (L, I) | | 0.000201 |
| YGYTHISAGELLR (SEQ ID NO.71) | 1.300000e-08 | (L, I) | | 0.000201 |
| TVNELQNLTATEVVVPR (SEQ ID NO.72) | 6.600000e-08 | (A, T) | | 0.000204 |
| TILDQHGQYPIWMNQR (SEQ ID NO.73) | 1.000000e-07 | (L, I) | | 0.000201 |
| AVFVDLEPTVIDDIR (SEQ ID NO.74) | 1.300000e-07 | (E, D) | | 0.000197 |
| AAELLANSLATAGDGLIELR (SEQ ID NO.75) | 1.700000e-07 | (I, L) | | 0.000199 |

Further, column "support" contains cell-line matched sequencing support; showing whether such variant was observed in sequencing. It can be seen that many of statistically significant results are not supported by sequencing. However, they have also low $P_{max}$ and can be rejected. The only two sequencing supported results in the example have $P_{max}= 1$.

*Determining identity - Maximal probability*

The criterium for determination of identity used here was maximal spectral match and $P_{max}= 1$.

*Results - Maximal probability*

*Identified variants*

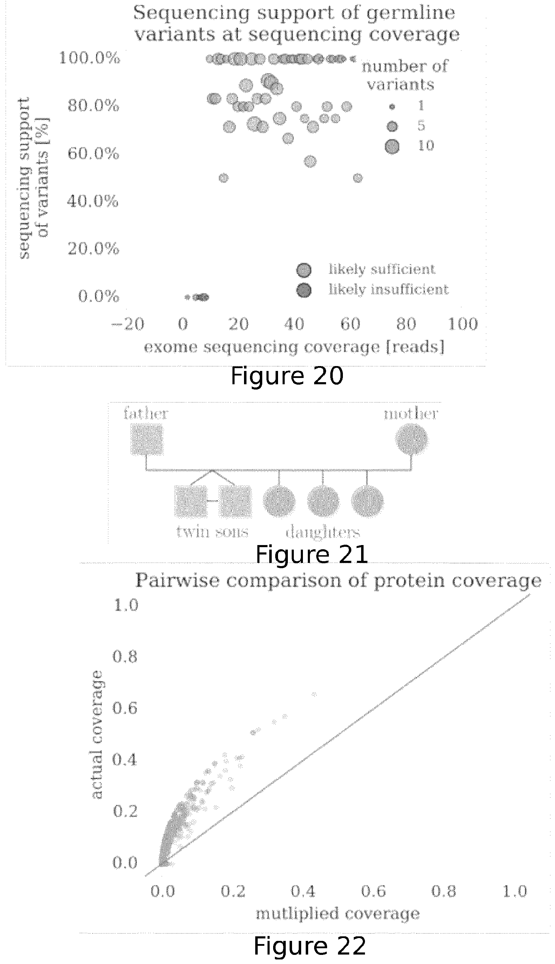

The method was used for identification of variants in human family members (Fig. 21). The following table contains numbers of identified variant peptides and their sequencing support (evaluated against exome sequencing), separately for each family member.

| family member | germline variants [a] | germline variants [b] | exome support |
|---|---|---|---|
| father | 38 | 30 (78.9 %) | 100.0 % |
| mother | 55 | 42 (76.4 %) | 91.3% |
| daughter 1 | 57 | 43 (75.4 %) | 91.5 % |
| daughter 2 | 61 | 53 (86.9 %) | 93.0 % |
| daughter 3 | 64 | 53 (82.8 %) | 94.6 % |
| son 1 (twin) | 35 | 24 (68.6 %) | 100.0 % |
| son 2 (twin) | 42 | 36 (85.7 %) | 94.7 % |
| average | 50.3 | 40.1 (79.8 %) | 95.0 % |

Note that the exome sequencing of particular sample was not used in construction of the global database. The evaluation of sequencing support against exome sequencing is most meaningful for germline variants as those are always present in substantial proportion.

*Comparison with proteome from translated exome*

The previous table also shows comparison of number of identified variants, if knowledge of exome sequencing was used to create proteome with all variants. In such case, germline variants were based on exome sequencing in a following way: variant was found in at least one parent and one kid. The results suggest that even if sequencing of sample is available, its benefits are limited as around 80% of germline variants are identified with the use of global nucleotide database (at around 95% of sequencing correspondence).

*Incompleteness of exome sequencing support*

In some cases when sequencing support is evaluated, the results might contain some of correct, but sequencing unsupported interpretation. This is because some variants are likely not sequencing supported because of low sequencing coverage of surrounding area, as illustrated on Fig. 20. For that reason, in previous comparison areas with coverage lower than 10 reads were removed from comparison.

**Example 3 - Authentication of cell lines**

This example shows utilization of claimed method for identification of cell line. The analysis is performed on publicly available data of NCI60 panel (Gholami et al. (2013) Cell Reports, 4(3): 609-620). Variants were identified as in previous example (the system architecture on Fig. 19). For establishment of genetic origin, only variants of high population frequency, (above 1% as specified in dbSNP) were considered; variants of this kind are likely germline variants, are easy to identify (not many more likely interpretations a priori, and statistical significance of E-Value < 0.1 is often enough) and are suitable for identification of origin.

*Matching to database of origins*

The identification of origin was performed against NCI60 exome database (Shankavaram et al. (2009) BMC Genomics, 10(1):277) and it was assumed that the true origin is within considered origins (thus within NCI60 exome database).

The process of matching of database of origins is further illustrated on a specific sample (P0001751) from NCI60 proteomes. An excerpt of results of variant peptide identification restricted to polymorphic peptides is shown in the following table:

| sequence | candidate SNVs | population frequency |
|---|---|---|
| G[R→P]EVDVNIPK (SEQ ID NO.76) | [(chr11, 62298073, 62298073, G, C)] | 0.01338 |

(continued)

| sequence | candidate SNVs | population frequency |
|---|---|---|
| EIENIQTY[Q→R] (SEQ ID NO.77) | [(chr4, 186379734, 186379734, A, G)] | 0.2133 |
| GAAGSGD[A→S]AAAAEWIR (SEQ ID NO.78) | [(chr19, 460641, 460641, G, T)] | 0.02097 |
| TILTLTGVSTL[G→R] (SEQ ID NO.79) | [(chr16, 2263835, 2263835, G, A), (chr16, 2263835, 2263835, G, C)] | 0.01058 |
| IIIE[Y→D]LLEATR (SEQ ID NO.80) | [(chr12, 120575548, 120575548, T, G)] | 0.9994 |
| GS[S→]GDRPEASMTPDAK (SEQ ID NO.81) | [(chr16, 89927150, 89927150, C, T)] | 0.03235 |
| GLVEPVN[V→M](Ox)VDNGDGTHTVTYTPSQEGPYM (Ox)VSVK (SEQ ID NO.82) | [(chr3, 58118554, 58118554, G, A)] | 0.6082 |
| VGEAGLLSV[D→N]C(Carb) SEAGPGALGLEAVSDSGTK (SEQ ID NO.83) | [(chr3, 58109161, 58109161, G, A)] | 0.5461 |
| QVVSAVT[T→A]LVEAAER (SEQ ID NO.84) | [(chr8, 144942902, 144942902, A, G)] | 0.6853 |

Polymorphic peptides were used to calculate match with exome sequencing data and used for calculation of probability of correct determination of origin, with an excerpt of results illustrated in the following table:

| cell line | product of matching | number of matching | probability |
|---|---|---|---|
| PR:PC 3 | 3.447000e-13 | 24 | 9.999987e-01 |
| CNS:SNB 19 | 5.679920e-07 | 17 | 6.068739e-07 |
| LE:SR | 1.853638e-06 | 19 | 1.859584e-07 |
| ME:MALME 3M | 2.686513e-06 | 18 | 1.283074e-07 |
| RE:ACHN | 4.735100e-06 | 17 | 7.279668e-08 |
| CNS:U251 | 5.081215e-06 | 18 | 6.783801e-08 |
| OV:OVCAR 8 | 6.737296e-06 | 14 | 5.116289e-08 |

The data indicates that the most likely cell line within considered origins is PR:PC 3. In the metadata of P0001751, it can be seen that the cell line is claimed as RE:SN12C, therefore it can be concluded that it is likely, that the cell line is mislabeled.

The procedure was further performed over all proteomes in the dataset; the results are visualized in the following table and point out two likely mislabeled cell lines:

| sample | Claimed cell line | Best matching cell line | Claimed: SNPs | Best: SNPs | Best: Error Prob. |
|---|---|---|---|---|---|
| P001891 | ME:UACC 62 | ME:UACC 62 | 41 | 41 | $2.14 \cdot 10^{-12}$ |
| P003198 | LC:NCI H23 | LC:NCI H23 | 26 | 26 | $2.52 \cdot 10^{-12}$ |
| P003381 | LC:NCI H460 | LC:NCI H460 | 42 | 42 | $7.86 \cdot 10^{-11}$ |
| P003207 | RE:ACHN | RE:ACHN | 32 | 32 | $4.41 \cdot 10^{-8}$ |
| P0001568 | CO:HCC 2998 | CO:HCC 2998 | 35 | 35 | $2.63 \cdot 10^{-7}$ |
| **P0001751** | **RE:SN12C** | **PR:PC 3** | **15** | **24** | **$1.28 \cdot 10^{-6}$** |
| **P003820** | **CO:KM12** | **CO:SW 620** | **32** | **41** | **$2.29 \cdot 10^{-6}$** |
| P001897 | ME:UACC 257 | ME:UACC 257 | 31 | 31 | $2.64 \cdot 10^{-6}$ |

(continued)

| sample | Claimed cell line | Best matching cell line | Claimed: SNPs | Best: SNPs | Best: Error Prob. |
|---|---|---|---|---|---|
| P001561 | CNS:SNB 75 | CNS:SNB 75 | 33 | 33 | $7.56 \cdot 10^{-6}$ |

Identification of mislabeled cell lines is of high importance, because it prevents propagation of incorrectly drawn conclusions and contributes to reproducibility of science outputs.

**Example 4 - Identification of a person**

This example shows utilization of the method for identification of a person. The analysis is performed on in-house data of family of particular structure (Fig. 21).

*Matching to database of origins*

The example is analogous to matching of cell lines. The database of origins corresponds to sequencing database of family members. The same methods are used for assignment.

*Results*

The results of identification are shown in the following table:

| sample | Claimed person | Best matching person | Claimed: SNPs | Best: SNPs | Best: Error Prob. |
|---|---|---|---|---|---|
| FAMD3 | daughter 3 | daughter 3 | 81 | 81 | $1.89 \cdot 10^{-8}$ |
| FAMM | mother | mother | 67 | 67 | $1.56 \cdot 10^{-7}$ |
| FAMD2 | daughter 2 | daughter 2 | 62 | 62 | $6.27 \cdot 10^{-4}$ |
| FAMF | father | father | 41 | 41 | $2.01 \cdot 10^{-2}$ |
| FAMD1 | daughter 1 | daughter 1 | 64 | 64 | $7.38 \cdot 10^{-2}$ |
| FAMS1 | son 1 (twin) | son 1 (twin) | 35 | 35 | 0.25 |
| FAMS2 | son 2 (twin) | son 1 (twin) | 53 | 55 | 0.40 |

The only misidentification happened in case of monozygous twin and was indicated by high probability of error (0.4).

**Example 5 - Presence of genetic relationship between two individuals**

This example illustrates the utilization of the identified variants for determination of genetic relationship. For this purpose, variants were identified in blood lymphocytes of family members (Fig. 21) in proteomics data (the architecture of variant identification was done as in Fig. 19, with determination of identity as maximal agreement among candidate identities and $P_{max}= 1$).

*Calculating significance of match*

The calculation of significance of match is further illustrated here. Similarly as in identification of genetic origin, only variants more prevalent than 1% (as population frequency in dbSNP) were used for calculation of match (these variants are more likely germline variants).

For calculation of pairwise match, it is beneficial to establish tabular structure, which helps in organization of data. Small subset of such tabular structure is visualized in the following table:

| Chr | Ref | Alt | Start | p+ | gene | $Pm_a+ (v)$ | $Pm_b+ (v)$ | $k(Pm_a+ (v), Pm_b+ (v))$ | a:has | b:has |
|---|---|---|---|---|---|---|---|---|---|---|
| chr11 | G | T | 62292881 | 1.0000 | ENSG00000124942 | 0.389718 | 0.349291 | 0.294993 | | |
| chr12 | T | G | 53070120 | 0.8011 | ENSG00000167768 | 0.347364 | 0.446011 | 0.222365 | | |
| chr1 | T | G | 40533346 | 1.0000 | ENSG00000131236 | 0.225182 | 0.267302 | 0.213775 | X | X |
| chr1 | T | G | 40533314 | 1.0000 | ENSG00000131236 | 0.225182 | 0.267302 | 0.213775 | | |
| chr1 | T | G | 40533265 | 1.0000 | ENSG00000131236 | 0.225182 | 0.267302 | 0.213775 | | |
| chr1 | T | G | 40533319 | 1.0000 | ENSG00000131236 | 0.225182 | 0.267302 | 0.213775 | | |
| chr1 | T | G | 40533286 | 1.0000 | ENSG00000131236 | 0.225182 | 0.267302 | 0.213775 | | |

In the table, each line corresponds to particular variant. The "p+" refers to population frequency of a variant from database, and $Pm_a+(v)$ to multiplication of coverage of individual gene in sample *a* and population frequency. Analogously for *b*. The **k(Pm$_a$+(v)**, **Pm$_b$+(v))** refers to probability of its identification in both samples.

The calculation of significance of match was approximated by Binomial distribution with mean value of

$$k\left(\mathrm{P}^{m_a}+(v), \mathrm{P}^{m_b}+(v)\right).$$

(50)

The results of this method (the approximation by Binomial distribution) are further illustrated here:

| k | n | p* | p-value |
|---|---|---|---|
| 0 | 35433 | 0.000474 | 1.000000e+00 |
| 1 | 35433 | 0.000474 | 9.999999e-01 |
| ... | ... | ... | ... |
| 23 | 35433 | 0.000474 | 8.624331e-02 |
| 24 | 35433 | 0.000474 * | 5.665743e-02 |
| 25 | 35433 | 0.000474 | 3.596854e-02 |
| 26 | 35433 | 0.000474 | 2.208026e-02 |
| ... | ... | ... | ... |

*Results*

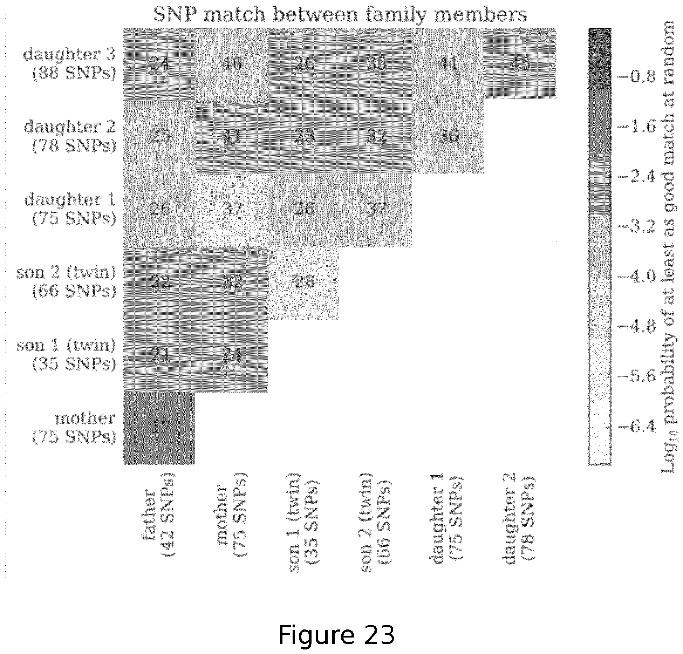

The method was further applied in pairwise manner to all family members (Fig. 21) and the heatmap (Fig. 23) shows results of at least as good match between them. The interest was thus in calculation of probability of at least as good match at random (43), here visualized as a color on the heatmap.

**Example 6 - Presence of a tumour in human**

This example illustrates embodiment for identification of tumour-specific circulating proteins in blood serum. In this example, publicly available data accessible on PRIDE (identifiers: PXD004624, PXD004625, PXD004626) were used for identification of mutant proteins. For identification of variants, the same method which corresponds to Fig. 19 was used.

*Determination of variant status*

*Selection of tumour-attributable mutations*

In this example, mutations attributable to tumour were assumed to be all somatic mutations identified.

*Results*

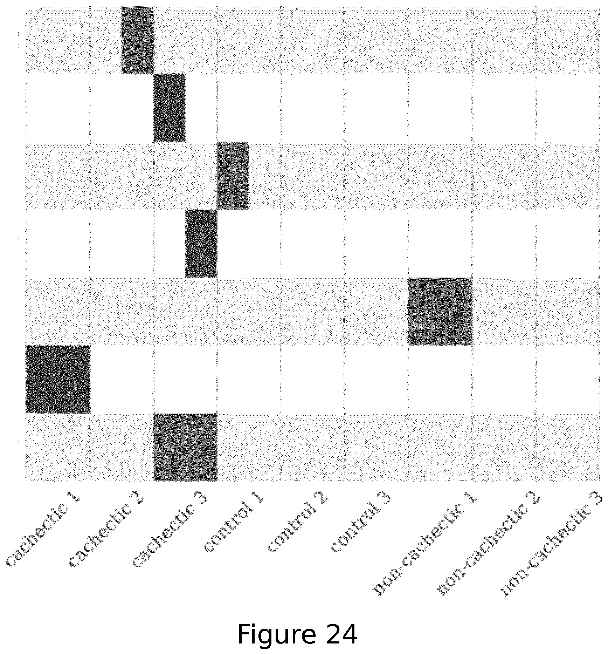

The results (Fig. 24) show presence of mutant peptides in melanoma cancer patients, with higher presence in advanced cachectic ones, lower presence in less advanced non-cachectic ones and almost none in controls. In this example, mutant peptides can be roughly associated with the presence of tumour and extent/stage of cancer.

**Example 7 - Identification of grafted peptides in xenograft model**

In this example, human reference and variant proteins are identified in blood serum from murine xenografts. Configuration of the experiment is based on Fig. 19, with difference in enumeration of candidate identities explained further.

*Obtaining candidate identities, Enumeration of candidate identities*

In enumeration of candidates, peptides are enumerated for both organisms (here, mouse and human), limiting condition being prior-like probability of $4 \cdot 10^{-6}$. Prior-like probabilities of peptides enumerated for human are multiplied (herein and in practice linearly scaled down) by relative difference of prevalence of human to mouse. The number is derived for particular experimental circumstances.

*Prevalence of peptides in xenografts*

The proportion in this example of $p = 0.25$ was estimated from homologous peptides and heterologous protein evidence, explained earlier. Tabular structure corresponding to homologous peptides and their heterologous protein evidence is illustrated here:

| homologous sequence | human proteins | mouse proteins | human evidence | mouse evidence |
|---|---|---|---|---|
| YIETDPANR (SEQ ID NO.85) | ENSP00000377882; ... | ENSMUSP00000028239; ... | | X |
| VGDTYERPK (SEQ ID NO.86) | ENSP00000346839; ... | ENSMUSP00000140816; ... | | X |
| DIFTGLIGPMK (SEQ ID NO.87) | ENSP00000420545; ... | ENSMUSP00000003714; ... | | X |
| SGIPIVTSPYQIHFTK (SEQ ID NO.88) | ENSP00000245907 | ENSMUSP00000024988 | X | X |
| KLWAYLTINQLLAER (SEQ ID NO.89) | ENSP00000351190; ... | ENSMUSP00000046530 | | X |
| RFDEILEASDGIMVAR (SEQ ID NO.90) | ENSP00000320171; ... | ENSMUSP00000128770; ... | | X |
| LLCNGDNDCGDQSDEANCRR (SEQ ID NO.91) | ENSP00000440113; ... | ENSMUSP00000066940; ... | | X |
| IHGILSNTHR (SEQ ID NO.92) | ENSP00000366460 | ENSMUSP00000110350; ... | | X |
| EVYGFNPEGK (SEQ ID NO.93) | ENSP00000382200; ... | ENSMUSP00000127147; ... | X | X |
| VNLGVGAYR (SEQ ID NO.94) | ENSP00000359539 | ENSMUSP00000026196 | | X |
| VQFELHYQEVK (SEQ ID NO.95) | ENSP00000351190; ... | ENSMUSP00000046530; ... | | X |

*Determination of identity*

Peptides with $P_{max}=1$ and maximal spectral agreement are retained.

*Results*

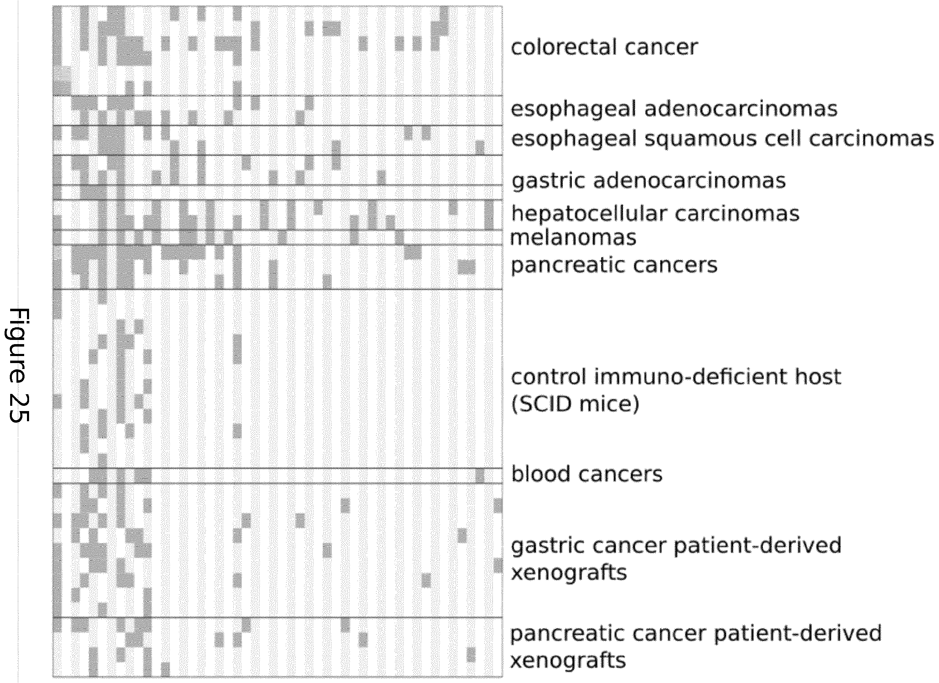

The identification method was used for identification of human protein biomarkers across wide range of cancer tissues transplanted to mice. The results (Fig. 25) show presence of human peptides and in general lack of such peptides in immunocompromised SCID mice, providing reliability of results.

**Example 8 - Identification of non-host organisms**

This example illustrates utilization of prevalence for diagnosis of mycoplasma in host organism. In this case, the prevalence of non-host organism is assumed to be unknown and thus refers to the more complicated situation as described earlier.

*Obtaining candidate identities*

For particular mass spectrum, peptides mapping exclusively to reference mycoplasmal peptides (among all organisms) and all human peptides (prior-like probability of $4 \cdot 10^{-6}$) were obtained. As previously described, mycoplasmal peptides were defined to be of strictly lower prevalence than any enumerated human peptide.

*Determining the identity*

Only mycoplasmal peptides of highest agreement and $P_{max}=1$ were retained.

*Results*

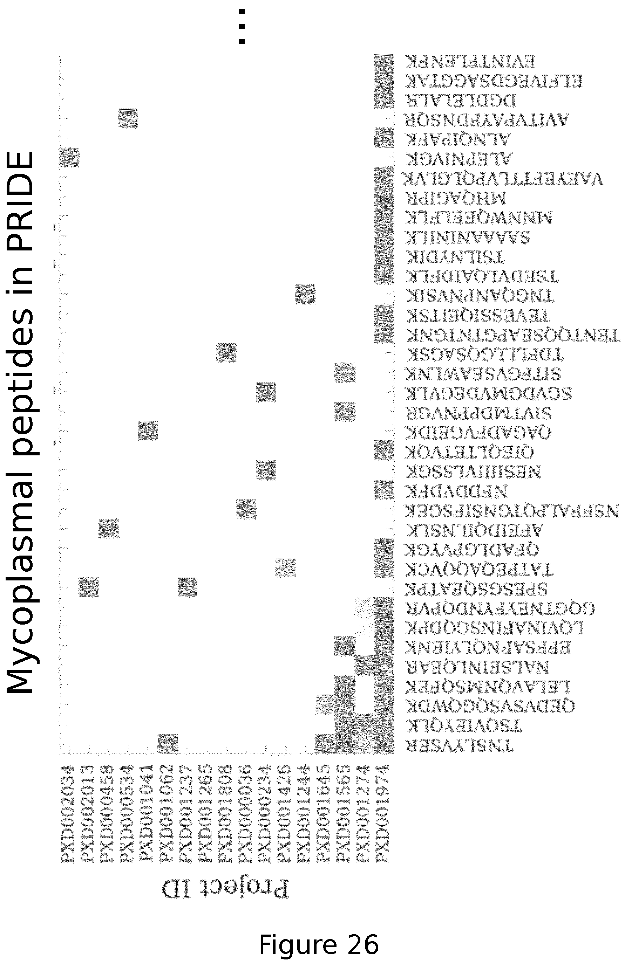

This identification approach was applied to subset of projects in PRIDE repository and results of diagnostics of mycoplasma are illustrated on Fig. 26 (SEQ ID NOs. 96-131).

**Example 9 - Use of isotopic labels for identification of mutations**

The following example illustrates utility of presence of both light and heavy isotopic forms using Stable isotope labeling with amino acids in cell culture (SILAC) of sample for identification of variants. The variant identification was performed as in previous examples (Fig. 19).

*Determining identity*

The additional criterium in this case is identification of both light and heavy forms of peptide of interest.

*Results*

The analysis of SILAC-pairing and its effect on sequencing support of variants was analyzed for two criteria (initial significance: E-Value $\leq$ 0.1) and after rejection ($P_{max}=1$). The results in the following table show that peptides which were identified in pairs have much higher sequencing support (when evaluated against sequencing of matching sample).

| | | Sequencing support | | | |
|---|---|---|---|---|---|
| | | Initial (E-Value < 0.1) | | After rejection (Pmax = 1) | |
| | | SILAC Paired | Single | SILAC Paired | Single |
| nonSNP | HCT116 | 2.3 % | 0.8 % | 45.5 % | 36.8% |
| | CCRF-CEM | 3.2 % | 0.7 % | 50.0 % | 29.4 % |
| SNP | HCT116 | 60.0% | 3.5% | 96.0 % | 100.0 % |
| | CCRF-CEM | 64.3% | 2.9% | 100.0 % | 82.4 % |

This difference was most remarkable in variants of low population frequency (more likely somatic mutations). Therefore, isotopic labels can be utilized to increase specificity of identification of somatic mutations.

**Example 10 - Identification of splicing variants**

*Obtaining candidate identities, Enumeration of candidate identities*

The scheme (Fig. 27) refers to enumeration, in which alternatively spliced proteins (and their prevalence) are constructed from reference exon-based protein models. This schema is in direct, unit-wise correspondence with generic enumeration (Fig. 5).

The enumeration for particular protein starts with reference exon-based protein model 2701, for which individual exons of corresponding gene are either present or not. Such a model can be represented by a binary vector representing presence of exon in the model. Different protein models 2702 are constructed by exon inclusion or exon exclusion events 2703 with associated effect on prevalence.

In some embodiments, prevalence might be expressed in prior-like probabilities and then exon inclusion, or exon exclusion are assigned probabilities of these events.

The enumeration process continues until limiting minimal prevalence condition 2704 is met. The protein models are transformed by concatenation of individual exons and translated into proteins 2705 with their associated prevalence, which then further constitutes the prevalence model 2706. Proteins constructed in this way might be used, for example, directly in top-down proteomics in identification, or proteins might be further digested for use in bottom-up proteomics.

**Example 11 - Identification of tumour, protein variants, correlation with clinical characteristics**

This example illustrates embodiment for identification of tumor size and associated disease stage. Herein, publicly available data from Clinical Proteomic Tumor Analysis Consortium, specifically TCGA Colorectal Cancer were used for identification of mutant proteins using the same method which corresponds to Fig. 19.

*Results*

The parameters of determination of somatic and germline variants are as follows. Germline variants are considered as follows: a variant is present in dbSNP (v. 147), or ExAC (version of ExAC compilation without TCGA) and is preferably of a population frequency higher than $1.10^{-4}$ (in any of dbSNP or ExAC)

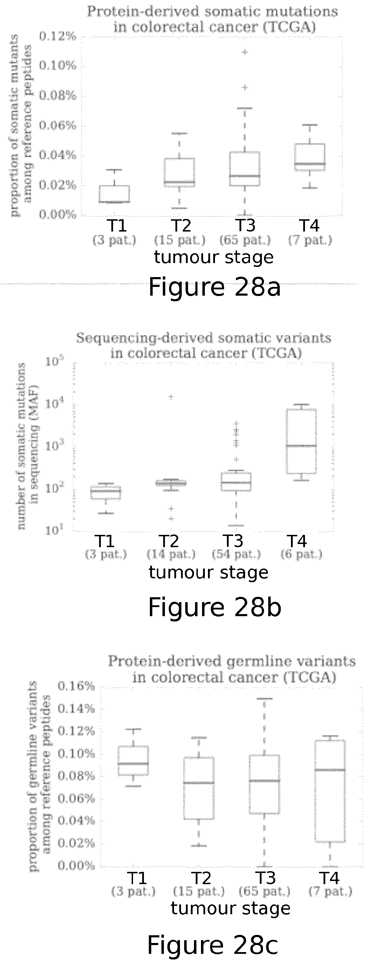

The results on Fig. 28 show the behavior of identified variants. The proportion of identified somatic mutant peptides among all reference peptides is visualized on Fig. 28a and shows clear increase in proportion of mutations with increasing tumour stage. Therefore, given particular reference measurement system, the increase in somatic mutations shows strong correlation to the tumour stage. Similar, but more pronounced effect can be seen when derived using nucleotide sequencing (Fig. 28b). Finally, the proportion of germline variants derived using proteomics does not show association with the tumour stage, showing that it is the effect of somatic mutations which is increased due to the higher tumour heterogeneity in more advanced stages.

**Claims**

1. A method for determination of identity of at least one entity from a mass spectrum of said at least one entity and optionally from additional data from chemical, physical, biochemical or biological analysis of said at least one entity, for each entity comprising the steps of:

    a) collecting analytical data from mass spectrum of the entity, and optionally collecting additional analytical data from a chemical, physical, biochemical or biological analysis of the entity,
    b) obtaining a plurality of candidate identities of the entity and obtaining the prevalences of said candidate identities of the entity, whereas for each candidate identity it applies that all candidate identities with a higher prevalence are included in the plurality of candidate identities;
    c) for each candidate identity of an entity, calculation of its score, said calculation involving at least prevalence of entity, or at least prevalence of entity and agreement with mass spectrum,
    d) determining the identity of an entity as the candidate identity with the score closest to the score which would correspond to the true identity of the entity.

2. The method according to claim 1, wherein in the step c), the calculation involves calculating maximal probability of candidate identity or calculating probability of candidate identity, optionally using Bayes' Theorem.

3. The method according to any one of the preceding claims, wherein in the step b) the value of prevalence is calculated based on at least one of population frequency of said entity, probability of modification of said entity in the environment, probability of modification of said entity during the analysis step.

4. The method according to any one of the preceding claims, wherein in the steps b) and c), the value of prevalence is expressed as prior probability or as prior-like probability.

5. The method according to any one of the preceding claims, wherein the entity is selected from a peptide, a protein, a lipid, a nucleic acid, a metabolite and a molecule having the molecular weight of up to 2000 mol/g.

6. The method according to any one of the preceding claims, wherein in the step b), the obtaining of the candidate entities and/or of prevalences of the candidate identities comprises enumeration which comprises the steps of:

    b.a) selecting initial candidate identities with initial prevalences;
    b.b) transferring said initial candidate identities into a base of candidate identities;
    b.c) producing new candidate identities by application of events to said base of candidate identities, and incorporating said new candidate identities into said base of candidate identity identities and continuing said producing unless a limiting condition is met;
    b.d) transforming the base of candidate identities obtained in step b.c) into candidate identities with associated prevalence.

7. The method according to claim 6, wherein said candidate identities are peptides; said prevalence is expressed as prior-like probability; said initial entities are N-terminally-cleaved linear subsequences of reference proteins; said applicable events comprise modification, substitution and cleavage; said limiting condition is minimal prior-like probability of given form of peptide; or
    wherein said candidate identities are proteins; said prevalence is expressed as prior-like probability; said initial entities are reference exon-based protein models; said applicable events comprise exon exclusion and exon inclusion; said limiting condition is minimal prior-like probability of exon-based model; said transformation of entities into hypotheses is concatenation of exons into protein-coding sequence and translation in silico.

8. The method according to any one of the preceding claims, wherein the entities are proteins, wherein the step of obtaining the candidate identities of an entity in step b) includes database search in database of peptide variants, and wherein the method is used for identification of mutant and polymorphic proteins from mass spectra of proteome, with alterations already observed globally on nucleotide level

9. The method according to any one of the preceding claims, wherein the entities are peptides, further comprising the steps of:
    e) matching of entities determined as polymorphic or germline peptides to database of origins,
    and wherein the method is used for authentication of cell lines or identification of a person from mass spectra of proteome.

10. The method according to any one of the preceding claims, wherein the entities are non-host peptides, wherein in the step b) the prevalence is expressed as prior or prior-like probability and prevalence of non-host peptides is scaled down according to prevalence of non-host organism,
and wherein the method is used for identification of non-host organism of known prevalence from mass spectra of proteome of host organism, for instance to identify microbial infection or colonization of the host.

11. The method according to any one of claims 6 to 8, wherein the entities are non-host peptides, wherein in the step b) in obtaining the candidate identities, peptides uniquely mapping to non-host organism are added to enumerated peptides of host organism and prevalence of non-host peptides is lower than of any host peptide,
and wherein the method is used for identification of non-host organism of unknown prevalence from mass spectra of proteome of host organism.

12. The method according to any one of the preceding claims, wherein the entities are donor peptides, wherein in the step b) the prevalence of donor peptides is scaled according to their prevalence among recipient peptides,
and wherein the method is used for identification of proteins originating from grafted tissue in recipient.

13. The method according to any one of the preceding claims, wherein the entities are peptides, the method further comprising the step of:
e) selecting somatic mutant peptides attributable to tumour,
wherein the method is used for identification of presence of a tumour from mass spectra of circulating proteins or estimation of tumour biological characteristics through increase in number of somatic mutations.

14. The method according to any one of the preceding claims, wherein the entities are peptides, the method further comprising the step of:
e) selection and quantification of polymorphic peptides attributable to donor,
wherein the method is used for monitoring tissue or organ grafting and early detection of transplant rejection from mass spectra of biological material of recipient.

15. The method according to any one of the preceding claims, wherein the entities are peptides, said method further comprising the steps of:
e) calculating significance of match between two individuals based on polymorphic peptides,
wherein the method is used for determination of presence of genetic relationship between two or more individuals from measured mass spectra of proteome.

Figure 1

Figure 2

Figure 3

Figure 4

Figure 5

**b**

| AA | p |
|---|---|
| A | $2 \cdot 10^{-3}$ |
| C | $2 \cdot 10^{-3}$ |
| D | $2 \cdot 10^{-3}$ |
| E | $2 \cdot 10^{-3}$ |
| F | $2 \cdot 10^{-3}$ |
| G | $2 \cdot 10^{-3}$ |
| H | $2 \cdot 10^{-3}$ |
| I | $2 \cdot 10^{-3}$ |
| K | 0.7 |
| L | $2 \cdot 10^{-3}$ |
| M | $2 \cdot 10^{-3}$ |
| N | $2 \cdot 10^{-3}$ |
| P | $2 \cdot 10^{-3}$ |
| Q | $2 \cdot 10^{-3}$ |
| R | 0.85 |
| S | $2 \cdot 10^{-3}$ |
| T | $2 \cdot 10^{-3}$ |
| V | $2 \cdot 10^{-3}$ |
| W | $2 \cdot 10^{-3}$ |
| Y | $2 \cdot 10^{-3}$ |

**a**

modifications $m_j$ of residue $a_i$ with probabilities $p_j$

$p_{cl}$ prob. of cleavage *after* residue $a_k$

**c**

| modification | p |
|---|---|
| M(Oxidation) | 0.3 |
| T(Methyl) | $1 \cdot 10^{-2}$ |
| S(Methyl) | $1 \cdot 10^{-2}$ |

Figure 6

72

702

Cell lines
and associated variants

702.1

703

701

Variants

Humans
and associated variants

702.2

Origin

Entities and
associated variants

702.3

Figure 7

Population

801

802

Entity

Entity

804

803

Variants

Variants

805

Correspondence

806

Figure 8

Figure 9

Interpretations of MS/MS spectrum
(545.780 Th, charge: 2)

% Propionyl
* Oxidation

relative intensity

mass to charge [Th]

Figure 10

Spectral match of true interpretations

Figure 11

Spectral match of random interpretations

Figure 12

Figure 13

Figure 14

Figure 15

Figure 16

Figure 17

Figure 18

Figure 19

Figure 20

Figure 21

Figure 22

Figure 23

Figure 24

Figure 25

Figure 26

Project ID

Mycoplasmal peptides in PRIDE

Figure 27

Figure 28a

Figure 28b

Figure 28c

**Europäisches Patentamt**
**European Patent Office**
**Office européen des brevets**

# EUROPEAN SEARCH REPORT

Application Number

EP 18 18 4710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 047 107 A2 (MICROMASS LTD [GB]) 25 October 2000 (2000-10-25) * CLAIMS 1-30; * | 1-15 | INV. G01N33/68 |
| X | M HIROSAWA ET AL: "MASCOT: multiple alignment system for protein sequences based on three-way dynamic programming.", BIOINFORMATICS, vol. 9, no. 2, 1 April 1993 (1993-04-01), pages 161-167, XP055579023, GB ISSN: 1460-2059 * the whole document * | 1-15 | |
| X | WO 2017/114943 A1 (VITO NV [BE]; UNIV ANTWERPEN [BE]) 6 July 2017 (2017-07-06) * claims 1-14; * | 1-15 | |
| X | EP 2 450 815 A1 (CONSEJO SUPERIOR INVESTIGACION [ES]) 9 May 2012 (2012-05-09) * claims 1-18; * | 1-15 | |
| X | WO 2008/151140 A2 (UNIV CALIFORNIA [US]; KIM SANGTAE [US] ET AL.) 11 December 2008 (2008-12-11) * claims 1-14; fig. 1-19; * | 1-15 | TECHNICAL FIELDS SEARCHED (IPC) G01N |
| X | US 2008/300795 A1 (SADYGOV ROVSHAN GOUMBATOGLU [US] ET AL) 4 December 2008 (2008-12-04) * claims 1-20; * | 1-15 | |
| X | WO 2005/057208 A1 (PROLEXYS PHARMACEUTICALS INC [US]; ASKOVIC SRDJAN [US] ET AL.) 23 June 2005 (2005-06-23) * claims 1-29; fig 1-3; * | 1-15 | |

The present search report has been drawn up for all claims

| Place of search | Date of completion of the search | Examiner |
|---|---|---|
| Munich | 2 May 2019 | Pilch, Bartosz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 18 4710

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2019

| Patent document cited in search report | | Publication date | Patent family member(s) | | Publication date |
|---|---|---|---|---|---|
| EP 1047107 | A2 | 25-10-2000 | CA | 2303761 A1 | 06-10-2000 |
| | | | DE | 60026452 T2 | 10-08-2006 |
| | | | EP | 1047107 A2 | 25-10-2000 |
| | | | EP | 1688987 A1 | 09-08-2006 |
| | | | US | 6489608 B1 | 03-12-2002 |
| WO 2017114943 | A1 | 06-07-2017 | JP | 2019505780 A | 28-02-2019 |
| | | | US | 2019018928 A1 | 17-01-2019 |
| | | | WO | 2017114943 A1 | 06-07-2017 |
| EP 2450815 | A1 | 09-05-2012 | DK | 2450815 T3 | 11-11-2013 |
| | | | EP | 2450815 A1 | 09-05-2012 |
| | | | ES | 2432677 T3 | 04-12-2013 |
| | | | US | 2012191685 A1 | 26-07-2012 |
| | | | WO | 2011000991 A1 | 06-01-2011 |
| WO 2008151140 | A2 | 11-12-2008 | US | 2010179766 A1 | 15-07-2010 |
| | | | WO | 2008151140 A2 | 11-12-2008 |
| US 2008300795 | A1 | 04-12-2008 | CA | 2632829 A1 | 01-12-2008 |
| | | | US | 2008300795 A1 | 04-12-2008 |
| WO 2005057208 | A1 | 23-06-2005 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SIELAFF et al.** *Journal of Proteome Research,* 2017, vol. 16 (11), 4060-4072
- **GHOLAMI et al.** *Cell Reports,* 2013, vol. 4 (3), 609-620
- **SHANKAVARAM et al.** *BMC Genomics,* 2009, vol. 10 (1), 277